# EUROPÄISCHE PATENTSCHRIFT

(11) **EP 3 426 601 B1**
(45) Veröffentlichungstag und Bekanntmachung des Hinweises auf die Patenterteilung: **06.05.2020**
(21) Anmeldenummer: 17709411.7
(22) Anmeldetag: 06.03.2017
(51) Int. Cl.: C01C 1/04, B01J 19/24, C07C 1/04, C07C 1/12

(54) **VERFAHREN SOWIE ANLAGE ZUR HERSTELLUNG EINES PRODUKTGASES UNTER WECHSELNDEN LASTBEDINGUNGEN**
METHOD AND SYSTEM FOR PRODUCING A PRODUCT GAS UNDER CHANGING LOAD CONDITIONS
PROCÉDÉ ET INSTALLATION POUR LA PRODUCTION D'UN PRODUIT GAZEUX SOUS DES CONDITIONS DE CHARGE VARIABLES

(30) Priorität: 08.03.2016 DE 102016203753
(43) Veröffentlichungstag der Anmeldung: 16.01.2019
(73) Patentinhaber: thyssenkrupp Industrial Solutions AG, 45143 Essen (DE); thyssenkrupp AG, 45143 Essen (DE)
(72) Erfinder: KOLBE, Bärbel, 58452 Witten (DE); ROOSEN, Christoph, 52146 Würselen (DE); JOHANNING, Joachim, 46045 Oberhausen (DE); SCHULTE BEERBÜHL, Simon, 44229 Dortmund (DE); SCHULTMANN, Frank, 76275 Ettlingen (DE)
(74) Vertreter: thyssenkrupp Intellectual Property GmbH
(86) Internationale Anmeldenummer: PCT/EP2017/055120
(87) Internationale Veröffentlichungsnummer: WO 2017/153304

(56) Entgegenhaltungen:
- EP-A1- 2 610 001
- CA-A1- 2 790 545
- US-A- 4 215 099
- US-A- 4 341 737
- US-A- 4 411 877
- Max Appl ET AL: "Ammonia, 2. Production Processes" In: "Ullmann's Encyclopedia of Industrial Chemistry", 8. November 2012 (2012-11-08), Wiley-VCH, Weinheim, XP055359081, ISBN: 978-3-527-30673-2 DOI: 10.1002/14356007.o02_o11, Absatz [6.3.1]; Abbildungen 30,41-44

## Beschreibung

Die vorliegende Erfindung betrifft ein Verfahren zur Synthese eines Produktgases umfassend die Schritte:
(a) Bereitstellen eines ersten Eduktgases;
(b) Bereitstellen eines zweiten Eduktgases;
(c) Vereinen des ersten Eduktgases und des zweiten Eduktgases unter Erhalt eines Gasgemischs A umfassend erstes Eduktgas und zweites Eduktgas;
(d) Vereinen des Gasgemischs A mit einem im Kreislauf geführten Gasgemisch unter Erhalt eines Gasgemischs B umfassend erstes Eduktgas, zweites Eduktgas und Produktgas;
(e) Umsetzen von in Gasgemisch B enthaltenem ersten Eduktgas und zweiten Eduktgas an einem Katalysator zu Produktgas unter Erzeugen eines an Produkt-gas angereicherten Gasgemischs C;
(f) Ausschleusen von Produktgas aus dem an Produktgas angereicherten Gasgemisch C unter Erzeugung eines an Produktgas verarmten Gasgemischs D; und
(g) Rückführen zumindest eines Teils des an Produktgas verarmten Gasgemischs D als im Kreislauf geführtes Gasgemisch zu Schritt (d);
wobei der in Schritt (a) bereitgestellte Volumenstrom des ersten Eduktgases und/oder der in Schritt (b) bereitgestellte Volumenstrom des zweiten Eduktgases und/oder der in Schritt (f) ausgeschleuste Volumenstrom des Produktgases während des Verfahrens variiert wird, weiterhin umfassend
- das vorübergehende Vermindern der pro Zeiteinheit gebildeten Menge des Produktgases während eines ersten Zeitintervalls durch Verringern des ersten Eduktgases und/oder des in Schritt (b) bereitgestellten Volumenstroms des zweiten Eduktgases und/oder des in Schritt (f) ausgeschleusten Volumenstroms des Produktgases; und
- des Erhöhen der pro Zeiteinheit gebildeten Menge des Produktgases während eines zweiten Zeitintervalls durch Erhöhen des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases.

Gegenstand der vorliegenden Erfindung ist weiterhin eine Anlage zur Herstellung eines Produktgases aus einem ersten Eduktgas und einem zweiten Eduktgas, umfassend die folgenden miteinander in Wirkverbindung stehenden Komponenten:
- eine erste Einlassvorrichtung konfiguriert zur Bereitstellung des ersten Eduktgases;
- eine zweite Einlassvorrichtung konfiguriert zur Bereitstellung des zweiten Eduktgases;
- einen Reaktor konfiguriert zur Synthese eines Produktgases aus dem ersten Eduktgas und dem zweiten Eduktgas, zur Erzeugung eines an Produktgas angereicherten Gasgemischs C unter heterogener Gaskatalyse;
- eine Ausschleusevorrichtung konfiguriert zum Ausschleusen von Produktgas aus dem an Produktgas angereicherten Gasgemisch C unter Erzeugung eines an Produktgas verarmten Gasgemischs D; und
- eine Steuerungsvorrichtung, welche konfiguriert ist, die Vorrichtung in einen ersten Betriebszustand oder in einen zweiten Betriebszustand zu versetzen, wobei sich der erste Betriebszustand von dem zweiten Betriebszustand
   (i) in einem geringeren Volumenstrom des ersten Eduktgases, welches durch die erste Einlassvorrichtung bereitgestellt wird, und/oder in einem geringeren Volumenstrom des zweiten Eduktgases, welches durch die zweite Einlassvorrichtung bereitgestellt wird, und/oder in einem geringeren Volumenstrom des Produktgases, welches durch die Ausschleusevorrichtung ausgeschleust wird,
      und/oder
   (ii) in einer geringeren pro Zeiteinheit gebildeten Menge des Produktgases unterscheidet.

Das Verfahren gemäß der vorliegenden Erfindung sieht vor, dass bei der Synthese eines Produktgases aus einem ersten Eduktgas und einem zweiten Eduktgas Produktgas ausgeschleust und nicht umgesetztes Eduktgas im Kreislauf geführt wird, wobei der Volumenstrom des ersten Eduktgases, des zweiten Eduktgases und/oder des ausgeschleusten Produktgases während des Verfahrens variiert wird. Auf diese Weise kann die pro Zeiteinheit gebildete Menge an Produktgas vorübergehend gedrosselt (Teillast-Betrieb) oder dessen Synthese vorübergehend vollständig eingestellt (Stand-by-Betrieb) werden, ohne dass die Anlage, in der das Verfahren durchgeführt wird, vollständig heruntergefahren werden muss. So können temporäre Engpässe bei der Verfügbarkeit von Energie bzw. von Eduktgas überbrückt werden. Das Verfahren kann dann kurzfristig wieder auf optimierte Synthesebedingungen hochgefahren werden (Volllast-Betrieb), wenn die Verfügbarkeit von Energie bzw. von Eduktgas wieder hergestellt ist. Die beiden Eduktgase können beispielsweise Wasserstoff und Stickstoff, das Produktgas kann Ammoniak sein.

Der Anteil erneuerbarer Energien an der Stromversorgung steigt stetig. In Deutschland lag der Anteil Ende 2012 bereits bei ca. 23% und soll bis zum Jahr 2020 noch auf bis zu 35% weiter ansteigen. Auch weltweit wird erwartet, dass der Anteil der erneuerbaren Energien an der gesamten Stromerzeugung deutlich zunehmen wird. Dabei wird insbesondere der Stromerzeugung aus Wind und Sonnenkraft ein besonders hohes Wachstum zugesprochen.

Diese Energieformen zeigen jedoch die Herausforderung, dass sie starken zeitlichen Schwankungen unterliegen (sowohl stündlich als auch saisonal) und deren Einspeiseprognose fehlerbehaftet ist. Die Nachfrage nach Elektrizität unterliegt hingegen deutlich anderen Schwankungen sowie ebenfalls Prognosefehlern. Häufig fallen Zeiten mit dem höchsten Verbrauch nicht zusammen mit Zeiten der höchsten Stromerzeugung aus erneuerbaren Energien.

Neben dieser stark temporären Erzeugung wird Strom aus Wind und Sonnenkraft auch regional in sehr unterschiedlichem Maße erzeugt, da sie von regionalen Gegebenheiten wie z.B. Windverhältnissen und Sonneneinstrahlung abhängen. Eine stark intermittierende Stromerzeugung führt einerseits zu starken Preisschwankungen im Strommarkt und zum anderen werden die Anforderungen an konventionelle Kraftwerke zum Ausgleich der temporären Schwankungen und der Netze zum Ausgleich der regionalen Differenzen immer größer. Stark regionale Unterschiede in der Stromerzeugung führen zu einer dauerhaft stärkeren Belastung und Kapazitätsanforderung der Stromnetze, so dass diese entsprechend ausgebaut werden müssen.

Um diesen Herausforderungen zu begegnen, werden zukünftig vermehrt Stromspeicher sowie Flexibilisierungsmaßnahmen auf Angebots- und Nachfrageseite benötigt, um Schwankungen sowohl in der Stromerzeugung als auch im Strompreis auszugleichen und die Netze zu entlasten.

Die Herstellung von Ammoniak aus Stickstoff und Wasserstoff ist ein seit vielen Jahrzehnten etabliertes großtechnisches, katalytisches Verfahren. Da es sich um eine Gleichgewichtsreaktion handelt, kann in einem Durchlauf durch den Reaktor nur ein Teil des Stickstoffs und Wasserstoffs umgesetzt werden. Nach teilweisem Ausschleusen des Ammoniaks durch Kondensation wird der restliche, nicht umgesetzte Stickstoff und Wasserstoff zum Reaktor zurückgeführt, es handelt sich also um einen Kreislaufprozess. Das Ammoniak wird kondensiert und abgeschieden. Da die Reaktion exotherm ist, muss die Reaktionswärme abgeführt werden.

Bei konventionellen Verfahren zur Synthese von Ammoniak sind die Reaktoren üblicherweise als adiabate Mehrbett-Reaktoren ausgeführt, d.h. mehrere Katalysatorbetten sind hintereinander geschaltet. Man unterscheidet zwischen Reaktoren mit direkter Kühlung (Quench-Reaktoren) und Reaktoren mit indirekter Kühlung, um die aus der exothermen Reaktion resultierende gestiegene Gastemperatur wieder abzusenken. In Quench-Reaktoren wird die Temperaturregelung dadurch erreicht, dass ein Teil des Gasgemischs nach Abkühlung unter Umgehung zumindest des ersten Katalysatorbetts zwischen nachgeschalteten Katalysatorbetten zugeführt wird. Im Unterschied zu dem vorgewärmten Teil des Gasgemischs, das auf das erste Katalysatorbett strömt, sind die Quench-Ströme deutlich kälter. In Reaktoren mit indirekter Kühlung wird die Temperaturregelung dadurch erreicht, dass sich zwischen den Katalysatorbetten ein Wärmeüberträger befindet, mit dessen Hilfe die Reaktionswärme über ein Kühlmedium abgeführt wird.

Die konventionellen Verfahren zur Synthese von Ammoniak sind für einen kontinuierlichen und stationären Betrieb ausgelegt. Der Teillast-Betrieb solcher Anlagen ist nur in einem begrenzten Umfang möglich und führt immer zu deutlich steigenden spezifischen Energieverbräuchen. Ein vollständiges Ausschalten der Anlagen ist stets sehr aufwändig und zeitintensiv. Des Weiteren würden die extremen Temperatur- und Druckschwankungen zu einer deutlichen Reduzierung der Haltbarkeit der eingesetzten Apparate und Komponenten führen.

Um nun NH₃ über den Zwischenschritt der Wasserelektrolyse intermittierend zu erzeugen, sind neue technische Konzepte nötig, die auch starke Schwankungen in der Last der Anlage erlauben. Während die zur Erzeugung des Wasserstoffs eingesetzte Wasserelektrolyse hoch flexibel auf die intermittierende Stromgestehung aus Windkraft und Solarenergie reagieren kann, ist die Synthese von NH₃ aus Stickstoff und Wasserstoff deutlich langsamer.

WO 2012/037571 A2 offenbart ein Verfahren, bei dem ein Wasserstoffspeicher/Puffer zwischen der Elektrolyse und der Ammoniaksynthese genutzt wird, um kurzfristige Schwankungen in der Wasserstofferzeugung auszugleichen. Auch kann ein solcher Puffer genutzt werden, um kurze Zeitperioden zu überbrücken, in denen kein Wasserstoff produziert werden kann.

Um einen Wasserstoffspeicher nicht beliebig groß auslegen zu müssen, was zu sehr hohen Investitionen führen würde, werden technische Konzepte benötigt, die es erlauben, die Ammoniaksynthese im Teillast-Betrieb durchzuführen, ohne die Anlage übermäßig zu belasten und ohne die spezifischen Verbräuche stark zu erhöhen.

Bei der Erzeugung von Wasserstoff über eine Wasserelektrolyse können immer wieder Zeiten auftreten, an denen es für mehrere Tage oder Wochen nicht sinnvoll ist, Wasserstoff zu produzieren. Um dies auszugleichen, wäre ein sehr großer Puffer nötig, der jedoch nur selten vollständig genutzt wird, so dass die nötigen Investitionen unverhältnismäßig wären. Um dies zu umgehen, sind technische Konzepte nötig, um die Ammoniaksynthese schnell in einen (materialschonenden) Stand-by Modus zu fahren und auch wieder schnell anzufahren.

Diese Betrachtungen gelten in ähnlicher Form für weitere Produkte, die aus Synthesegas hergestellt werden, z.B. für die Herstellung von Methanol.

Aus der EP 2 589 574 B1 ist ein Verfahren zur Lastregelung einer Ammoniakanlage bekannt, bei dem die Ammoniakanlage einen Hochdruck-Ammoniaksynthesekreis bzw. -kreislauf umfasst, der mindestens einen Ammoniakreaktor aufweist, in dem ein Frischgas, umfassend eine Wasserstoffzufuhr und eine Stickstoffzufuhr, bei einem Synthesedruck in Ammoniak umgewandelt wird, wobei der Kreis auch eine Spülleitung umfasst, die so angeordnet ist, ein Spülgas, das Inertgase enthält, aus dem Kreis abzuziehen, wobei das Verfahren auch einen Teillastbetrieb der Anlage vorsieht. Während dieses Teillastbetriebs wird der Ammoniaksynthesekreis beim Synthesedruck betreiben und der Spülgasstrom wird verringert, wobei das Verhältnis zwischen der molaren Durchflussmenge des Spülgases und der molaren Durchflussmenge des Frischgases auf einen Wert verringert wird, der kleiner als ein Nennwert des Verhältnisses ist, das verwendet wird, wenn die Ammoniakanlage bei Nennlast betrieben wird. Nachteilig an diesem Konzept ist jedoch, dass es bei geringen Inertgasanteilen im Frischgas nur zu einer sehr langsamen Erhöhung des Inertgasanteils im Synthesekreislauf kommt. Daher erlaubt ein solches Konzept nur eine sehr langsame Absenkung und Erhöhung des Teillastbereiches.

Der Erfindung liegt die Aufgabe zugrunde, ein alternatives Verfahren und eine alternative Anlage zur Herstellung von Produkten durch heterogene Gaskatalyse bereitzustellen, welche insbesondere eine schnelle Laständerung, d.h. ein schnelles Umstellen von Vollastbetrieb auf Teillastbetrieb und umgekehrt erlauben.

Diese Aufgabe wird durch ein Verfahren mit den Merkmalen des Anspruchs 1 bzw. eine Anlage mit den Merkmalen des Anspruchs 13 gelöst.

Die beschriebene Variation der Volumenströme versetzt das erfindungsgemäße Verfahren vorzugsweise in unterschiedliche Zustände, bei denen pro Zeiteinheit unterschiedliche Mengen an Produktgas gebildet werden, welche erfindungsgemäß bevorzugt als *"Stand-by-Betrieb", "Teillast-Betrieb"* und *"Volllast-Betrieb"* bezeichnet werden. Im Hinblick auf die pro Zeiteinheit gebildete Menge an Produktgas verhalten sich die Zustände gemäß folgender Rangfolge: Stand-by-Betrieb < Teillast-Betrieb < Volllast-Betrieb. Bevorzugt werden bei dem erfindungsgemäßen Verfahren wenigstens zwei dieser Zustände nacheinander durchlaufen. Ein Fachmann erkennt, dass ausgehend vom Volllast-Betrieb wenigstens für ein kurzes Zeitintervall ein Teillast-Betrieb durchlaufen werden muss, um das Verfahren schließlich in den Stand-by-Betrieb zu versetzen, und umgekehrt. Die unterschiedliche pro Zeiteinheit gebildete Menge an Produktgas kann erfindungsgemäß auf unterschiedliche Maßnahmen sowie deren Kombination erreicht werden.

Das erfindungsgemäße Verfahren ist dazu geeignet, temporär in unterschiedlicher Menge verfügbare Energie, z.B. elektrische Energie aus regenerativen Quellen, in Form von chemischer Energie zu speichern. Beispielsweise kann Wasser durch Elektrolyse in Wasserstoff und Sauerstoff gespalten und der so gewonnene Wasserstoff bei späterem Bedarf wieder verstromt werden. Die zwischenzeitliche Speicherung von Wasserstoff (erstes Eduktgas) kann erfindungsgemäß durch Umsetzung mit Stickstoff (zweites Eduktgas) zu Ammoniak (Produktgas) erfolgen. Das auf diese Art gewonnene Ammoniak kann bei Bedarf wieder in Wasserstoff und Stickstoff gespalten werden oder es kann auch direkt wieder durch Verbrennung von Ammoniak Strom gewonnen werden. Alternativ kann Ammoniak auch als regenerativ erzeugtes Produkt im Chemikalien- oder Düngemittelmarkt verkauft werden.

Das erfindungsgemäße Verfahren ermöglicht es, durch einen Teillast-Betrieb variierende Lasten in der Erzeugung von Eduktgas, z.B. Wasserstoff, auf die Synthese von Produktgas, z.B. Ammoniak, zu übertragen. Durch die gleichzeitige Kombination mit einem Puffer, z.B. einem Wasserstoffpuffer, kann ein solches System sehr schnell auf Änderungen in der Verfügbarkeit von Eduktgas (z.B. H₂ und N₂) reagieren und eine gewisse Zeit, in der kein Eduktgas erzeugt wird, über den Puffer ausgleichen. Die Gesamtflexibilität des Prozesses steigt auf diese Weise bei gleichzeitig geringem Pufferbedarf.

Eine bevorzugte Weiterbildung des Verfahrens sieht vor, dass das Ausschleusen des Produktgases in Schritt (f) durch Auskondensieren mittels eines Kondensators erfolgt und im ersten Zeitintervall die Austrittstemperatur des Kondensators erhöht wird. Durch die Erhöhung der Kondensationstemperatur bei Teillast erzielt man höhere Produktgehalte im Recycle-Strom Dies führt zu einer Verschiebung des Reaktionsgleichgewichts und zur Senkung der Partialdrücke der Eduktgase im Gasgemisch.

Im Rahmen des erfindungsgemäßen Verfahrens kann die vorgenannte Maßnahme der Erhöhung der Austrittstemperatur des Kondensators grundsätzlich auch als alleinige Maßnahme bei einem Konverter mit nur einem Katalysatorbett umgesetzt werden. Bevorzugt erfolgt diese Maßnahme jedoch in Kombination mit einer Veränderung der Quenchströme bei Teillastbetrieb der Anlage. Gemäß Simulationsrechnungen ist mit diesen Maßnahmen eine Absenkung der Last bis auf 20% der Nennlast möglich (üblich sind 60% der Nennlast).

Bei einer mindestens teilweisen Umgehung des Katalysators ist ein schnelles Fahren in den Stand-by Betrieb und zurück in den Normalbetrieb möglich, ohne den Konverter starken Temperaturwechsel-Belastungen auszusetzen.

Die in der vorliegenden Erfindung vorgeschlagenen Maßnahmen dienen zur Einstellung eines geeigneten Temperaturprofils in der Reaktionszone bei Betrieb der Anlage in Teillast. Dies ist vorteilhaft bei einer Anlage zur Herstellung von Ammoniak durch Umsetzung von Stickstoff und Wasserstoff, wobei die vorliegende Erfindung jedoch nicht auf diese Reaktion beschränkt ist.

Erfindungsgemäß erfolgt eine Veränderung der Volumenströme der Eduktgase und/oder des ausgeschleusten Volumenstroms im Teillastbetrieb der Anlage, wobei es besonders vorteilhaft ist, wenn die gewünschte Konzentrationsänderung im Recyclestrom durch nicht-proportionale Änderung des ausgeschleusten Volumenstroms im Verhältnis zu den Eduktströmen erreicht wird.
Figur 1 illustriert schematisch eine bevorzugte Variante der erfindungsgemäßen Vorrichtung zur Durchführung des erfindungsgemäßen Verfahrens (Kreislaufprozess), wobei zur Vereinfachung nicht alle Wärmeüberträger, insbesondere kein Wärmeverbund zwischen den Prozessströmen, dargestellt sind.
Figur 2 illustriert schematisch eine bevorzugte Variante der erfindungsgemäßen Vorrichtung zur Durchführung des erfindungsgemäßen Verfahrens für den Stand-by-Betrieb zum Warmhalten des Synthesekreislaufs.
Figur 3 illustriert schematisch eine Variante der Ausführungsform gemäß Figur 2, bei welcher der separate Kreislauf für den Stand-by-Betrieb allerdings kleiner ist.
Figur 4 zeigt den relativen Anteil von Ammoniak (Produktgas) im thermodynamischen Gleichgewicht in Abhängigkeit von der Temperatur bei verschiedenen Drücken.
Figur 5 stellt einen möglichen Verlauf der erzeugten Menge Ammoniak im jeweiligen Katalysatorbett im Lastbereich von 30% bis 100% bezogen auf den Gesamtumsatz im Volllast-Betrieb dar.
Figur 6 zeigt das Verhältnis des relativen Anteils von Ammoniak (Produktgas) zur Temperatur für einen Dreibett-Quench-Reaktor (a) unter Volllast und (b) unter 30% Teillast. Die durchgezogenen Graphen betreffen jeweils eines der drei Katalysatorbetten.
Figur 7 illustriert einen Quench-Reaktor, bei dem im Stand-by-Betrieb das Gasgemisch zwischen der äußeren Hülle des Reaktors und den Katalysatorbetten geführt werden kann.

Das erfindungsgemäße Verfahren zeichnet sich bevorzugt durch mindestens zwei Zustände aus, welche während mindestens zwei aufeinander folgender, bevorzugt unmittelbar aufeinander folgender Zeitintervalle durchlaufen werden. Zum Zwecke der Beschreibung werden diese beiden Zeitintervalle nachfolgend als *"erstes Zeitintervall*" und als "*zweites Zeitintervall*" bezeichnet. Das erste Zeitintervall kann zeitlich vor oder nach dem zweiten Zeitintervall liegen. Vorzugsweise zeichnet sich der Zustand des Verfahrens während des ersten Zeitintervalls durch eine geringere Ausbeute an Produktgas aus als der Zustand des Verfahrens während des zweiten Zeitintervalls. Insbesondere bevorzugt betrifft der Zustand des Verfahrens während des ersten Zeitintervalls den Teillast-Betrieb und der Zustand des Verfahrens während des zweiten Zeitintervalls den Volllast-Betrieb.

Gemäß einer bevorzugten Weiterbildung der Erfindung ist mindestens ein drittes Zeitintervall vorgesehen, in dem der in Schritt (a) bereitgestellte Volumenstrom des ersten Eduktgases und/oder der in Schritt (b) bereitgestellte Volumenstrom des zweiten Eduktgases und/oder der in Schritt (f) ausgeschleuste Volumenstrom des Produktgases vollständig eingestellt wird. Dieses dritte Zeitintervall entspricht einem Stand-by-Betrieb der Anlage.

Beginn und Ende des ersten Zeitintervalls werden bevorzugt bestimmt durch eine im Vergleich zum zweiten Zeitintervall geringere Verfügbarkeit von Ressourcen, insbesondere von elektrischem Strom und/oder erstem Eduktgas und/oder zweitem Eduktgas. Das erste Zeitintervall beginnt bevorzugt zu dem Zeitpunkt, an dem die im Vergleich zum zweiten Zeitintervall geringere Verfügbarkeit von elektrischem Strom und/oder erstem Eduktgas und/oder zweitem Eduktgas einsetzt und endet bevorzugt an dem Zeitpunkt, an dem die Verfügbarkeit von elektrischem Strom und/oder erstem Eduktgas und/oder zweitem Eduktgas wieder vollständig hergestellt ist. Bevorzugt beginnt das zweite Zeitintervall an dem Zeitpunkt, an welchem das erste Zeitintervall endet. Bevorzugt ist somit das erste Zeitintervall über seine gesamte Dauer durch eine im Vergleich zum zweiten Zeitintervall geringere Verfügbarkeit von elektrischem Strom und/oder erstem Eduktgas und/oder zweitem Eduktgas gekennzeichnet.

Richten sich Anfang und Ende des ersten bzw. des zweiten Zeitintervalls nach der unterschiedlichen Verfügbarkeit von elektrischem Strom, so wird dieser bevorzugt durch erneuerbare Energien, insbesondere durch Photovoltaik und/oder durch Windkraft, erzeugt.

In einer besonders bevorzugten Ausführungsform ist die Verfügbarkeit des ersten Eduktgases, vorzugsweise Wasserstoff, ursächlich an die Verfügbarkeit des elektrischen Stroms gekoppelt. Bei dieser bevorzugten Ausführungsform wird das erste Eduktgas, vorzugsweise Wasserstoff, bevorzugt durch Elektrolyse gewonnen, wobei der bei der Elektrolyse eingesetzte elektrische Strom vorzugsweise durch erneuerbare Energien gewonnen wird, insbesondere durch Photovoltaik und/oder Windkraft. Ist die Verfügbarkeit von elektrischem Strom temporär eingeschränkt, so steht entsprechend auch weniger elektrischer Strom für die Elektrolyse von Wasser zu Verfügung und als Folge ist auch die Verfügbarkeit von Wasserstoff (erstes Eduktgas) temporär eingeschränkt.

Das zweite Zeitintervall ist erfindungsgemäß bevorzugt im Unterschied zum ersten Zeitintervall durch eine ausreichend hohe Verfügbarkeit von elektrischem Strom und/oder durch eine ausreichend hohe Verfügbarkeit von erstem Eduktgas, vorzugsweise Wasserstoff, und/oder zweitem Eduktgas gekennzeichnet, um das erfindungsgemäße Verfahren unter maximaler pro Zeiteinheit gebildeter Menge (d.h. bei maximalem Umsatz) durchführen zu können.

Bei allen erfindungsgemäßen bevorzugten Ausführungsformen, welche über ein erstes Zeitintervall und ein zweites Zeitintervall definiert werden, beträgt die jeweilige Dauer des Zeitintervalls unabhängig voneinander bevorzugt jeweils mindestens 30 Minuten, bevorzugter mindestens 1 Stunde, noch bevorzugter mindestens 2 Stunden, am bevorzugtesten mindestens 6 Stunden und insbesondere mindestens 12 Stunden.

Dient das erfindungsgemäße Verfahren beispielsweise der Synthese von Ammoniak aus Wasserstoff und Stickstoff und wird dafür der Wasserstoff durch Wasserelektrolyse bereitgestellt, für welche die benötigte Elektrizität durch Photovoltaik erzeugt wird, so können erstes und zweites Zeitintervall durch den Tag-Nacht-Zyklus vorgegeben sein. Das erste Zeitintervall umfasst dann bevorzugt die Nacht-Phase und das zweite Zeitintervall die Tag-Phase. Wird die für die Elektrolyse von Wasser benötigte Elektrizität hingegen durch Windkraft erzeugt, so können erstes und zweites Zeitintervall durch die Wetterlage vorgegeben sein. Das erste Zeitintervall umfasst dann bevorzugt die Flaute-Phase und das zweite Zeitintervall die Wind-Phase.

In einer bevorzugten Ausführungsform des erfindungsgemäßen Verfahrens werden der in Schritt (a) bereitgestellte Volumenstrom des ersten Eduktgases und/oder der in Schritt (b) bereitgestellte Volumenstrom des zweiten Eduktgases und/oder der in Schritt (f) ausgeschleuste Volumenstrom des Produktgases während des Verfahrens variiert, so dass
(i) während des ersten Zeitintervalls ein erster Volumenstrom und während des zweiten Zeitintervalls ein zweiter Volumenstrom des ersten Eduktgases bereitgestellt wird, welcher sich von dem ersten Volumenstrom unterscheidet; und/oder
(ii) während des ersten Zeitintervalls ein erster Volumenstrom und während des zweiten Zeitintervalls ein zweiter Volumenstrom des zweiten Eduktgases bereitgestellt wird, welcher sich von dem ersten Volumenstrom unterscheidet; und/oder
(iii) während des ersten Zeitintervalls ein erster Volumenstrom und während des zweiten Zeitintervalls ein zweiter Volumenstrom des Produktgases ausgeschleust wird, welcher sich von dem ersten Volumenstrom unterscheidet.

Bevorzugt werden jeweils sowohl der in Schritt (a) bereitgestellte Volumenstrom des ersten Eduktgases als auch der in Schritt (b) bereitgestellte Volumenstrom des zweiten Eduktgases gleichzeitig und bevorzugt auch gleichermaßen variiert.

In einer bevorzugten Ausführungsform des erfindungsgemäßen Verfahrens wird während des ersten Zeitintervalls in Schritt (a) ein geringerer Volumenstrom des ersten Eduktgases und/oder in Schritt (b) ein geringerer Volumenstrom des zweiten Eduktgases bereitgestellt und/oder in Schritt (f) ein geringerer Volumenstrom des Produktgases ausgeschleust als während des zweiten Zeitintervalls, welches auf das erste Zeitintervall folgt, oder welches dem ersten Zeitintervall vorausgeht.

Bevorzugt wird jeweils während des ersten Zeitintervalls in Schritt (a) sowohl ein geringerer Volumenstrom des ersten Eduktgases als auch in Schritt (b) ein geringerer Volumenstrom des zweiten Eduktgases bereitgestellt als während des zweiten Zeitintervalls, welches auf das erste Zeitintervall folgt, oder welches dem ersten Zeitintervall vorausgeht.

In einer bevorzugten Ausführungsform umfasst das erfindungsgemäße Verfahren
- das vorübergehende Vermindern der pro Zeiteinheit gebildeten Menge des Produktgases während des ersten Zeitintervalls durch Verringern (Teillast-Betrieb) oder vollständiges Einstellen (Stand-by-Betrieb) des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms an Produktgas; und anschließend, bevorzugt unmittelbar anschließend
- das Erhöhen der pro Zeiteinheit gebildeten Menge des Produktgases während des zweiten Zeitintervalls durch Erhöhen des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases. Bevorzugt wird die pro Zeiteinheit gebildete Menge des Produktgases dabei optimiert (Volllast-Betrieb).

Das vorübergehende Vermindern der pro Zeiteinheit gebildeten Menge im Teillast-Betrieb oder im Stand-by-Betrieb wird bevorzugt bestimmt durch eine geringere Verfügbarkeit von elektrischem Strom, der bevorzugt durch erneuerbare Energien, insbesondere Photovoltaik und/oder Windkraft, erzeugt wird, noch bevorzugter durch eine geringere Verfügbarkeit von erstem und/oder zweitem Eduktgas, noch bevorzugter durch eine geringere Verfügbarkeit von Wasserstoff, der bevorzugt durch Elektrolyse gewonnen wird.

Eine bevorzugte Ausführungsform des erfindungsgemäßen Verfahrens umfasst das Betreiben des Verfahrens im Teillast-Betrieb und anschließend im Volllast-Betrieb, und/oder umgekehrt.

Eine andere bevorzugte Ausführungsform des erfindungsgemäßen Verfahrens umfasst das Betreiben des Verfahrens im Stand-by-Betrieb, anschließend im Teillast-Betrieb und danach im Volllast-Betrieb, und/oder umgekehrt.

Auch ein alternierender Betrieb ist erfindungsgemäß möglich, z.B.
- Volllast-Betrieb -> Teillast-Betrieb -> Volllast-Betrieb -> Teillast-Betrieb etc; oder
- Volllast-Betrieb -> Teillast-Betrieb -> Stand-by-Betrieb -> Teillast-Betrieb -> Volllast-Betrieb -> Teillast-Betrieb-> Stand-by-Betrieb -> Teillast-Betrieb -> Volllast-Betrieb etc; oder
- Volllast-Betrieb -> Teillast-Betrieb -> Stand-by-Betrieb -> Teillast-Betrieb -> Volllast-Betrieb -> Teillast-Betrieb-> Volllast-Betrieb etc.

Der Volllast-Betrieb des erfindungsgemäßen Verfahrens umfasst bevorzugt das Betreiben bei optimierter pro Zeiteinheit gebildeter Menge des Produktgases, vorzugsweise während des zweiten Zeitintervalls. Bevorzugt erfolgt die Optimierung dahingehend, dass die Verfügbarkeit des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases keinen vermindernden Einfluss auf die pro Zeiteinheit gebildete Menge des Produktgases hat, sondern diese durch andere Faktoren limitiert wird, insbesondere die Maximalkapazität, welche die Anlage, auf der das erfindungsgemäße Verfahren durchgeführt wird, unter optimalen Bedingungen hat. Der Volllast-Betrieb umfasst somit grundsätzlich eine unbegrenzte Verfügbarkeit des in Schritt (a) bereitgestellten Volumenstroms des ersten Eduktgases und/oder des in Schritt (b) bereitgestellten Volumenstroms des zweiten Eduktgases und/oder eine unbegrenzte, d.h. praktisch nahezu vollständige Ausschleusung des Volumenstroms des Produktgases in Schritt (f).

Der Teillast-Betrieb des erfindungsgemäßen Verfahrens umfasst bevorzugt im Vergleich zum Volllast-Betrieb das vorübergehende Vermindern der pro Zeiteinheit gebildeten Menge des Produktgases durch Verringern des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases, vorzugsweise jeweils während des ersten Zeitintervalls.

In verschiedenen Fällen kann der Teillast-Betrieb jedoch nicht ausreichend sein, um das erfindungsgemäße Verfahren bei vorübergehend verminderter Verfügbarkeit an Ressourcen (z.B. elektrischer Strom, erstes Eduktgas, zweites Eduktgas, etc.) in einen Zustand zu versetzen, welcher keine vollständige Abschaltung bedeutet und dennoch bei Überwindung der verminderten Verfügbarkeit, d.h. der Wiederkehr der Ressourcen das ggf. schnelle Hochfahren auf Volllast-Betrieb ermöglicht. In diesen Fällen wird erfindungsgemäß bevorzugt die Synthese an Produktgas vorübergehend vollständig eingestellt (Stand-by-Betrieb).

Der Stand-by-Betrieb des erfindungsgemäßen Verfahrens umfasst bevorzugt im Vergleich zum Teillast-Betrieb das vorübergehende weitergehende Vermindern der pro Zeiteinheit gebildeten Menge des Produktgases durch Einstellen, d.h. vollständiges Unterbrechen, des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases, vorzugsweise jeweils während des ersten Zeitintervalls.

Durch den Stand-by-Betrieb können erfindungsgemäß insbesondere einerseits die Apparate vor zu starken Temperatur- und Druckschwankungen geschützt und andererseits ein schnelles An- und Abfahren der Anlage gewährleistet werden. Dabei können der Synthesekreislauf bzw. der Reaktor im Wesentlichen auf konstanter Reaktionstemperatur und konstantem Reaktionsdruck gehalten werden, um dadurch ein schnelles Anfahren sicher zu stellen und die Bauteile nicht unnötig zu belasten.

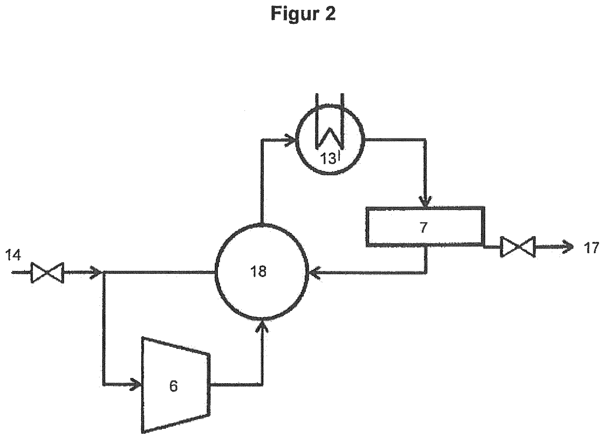

In einer besonders bevorzugten Ausführungsform (vgl. Figur 2) wird im Stand-by-Betrieb die Bereitstellung des ersten und zweiten Eduktgases abgeschaltet (Schritte (a) und (b)) und auch kein Produktgas mehr ausgeschleust (Schritt (f)). Ebenso wird auch kein Spülgas abgeführt. Der Kreislaufverdichter des Synthesekreislaufes wird hingegen weiterhin betrieben. Zum Ausgleich der Wärmeverluste wird bevorzugt eine zusätzliche Heizung in den Synthesekreislauf integriert. Ein Druckabfall durch ein Fortschreiten der Reaktion kann durch kurzzeitige Zufuhr von erstem Eduktgas, z.B. Wasserstoff, und/oder von zweitem Eduktgas, z.B. Stickstoff, ausgeglichen werden. Sollte eine Kühlung aufgrund einer zu starken Wärmeentwicklung durch Reaktion der verbleibenden Eduktgase nötig sein, so kann dies ebenfalls im Kreislauf realisiert werden. Die Heizung kann elektrisch oder mit einem Heizmedium, bevorzugt Dampf, ausgeführt werden.

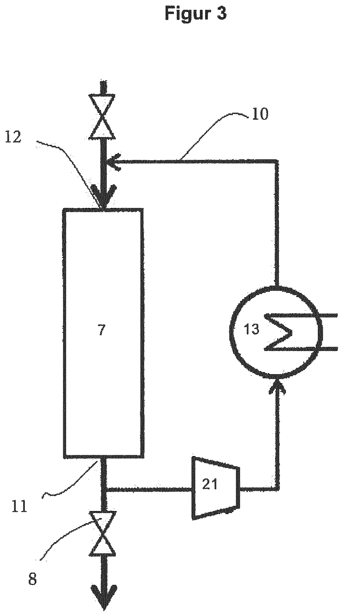

In einer anderen, besonders bevorzugten Ausführungsform (vgl. Figur 3) werden im Stand-by-Betrieb nicht der komplette Synthesekreislauf, Kreislaufverdichter, sowie eine zusätzliche Heizung zum Ausgleich der Wärmeverluste genutzt, sondern es wird ein separater kleinerer Kreislauf um den Reaktor genutzt. Aufgrund der geringeren Kreislaufgröße sind bei diesem Betrieb die Wärmeverluste geringer, so dass eine kleinere Zusatzheizung ausreicht, um eventuell auftretende Wärmeverluste auszugleichen. Auch die Druckverluste sind geringer, so dass ggf. ein Gebläse zum Zirkulieren des Gasstroms genügt. Ein Druckabfall durch ein Fortschreiten der Reaktion kann durch kurzzeitige Zufuhr von erstem Eduktgas, z.B. Wasserstoff, und/oder zweitem Eduktgas, z.B. Stickstoff, ausgeglichen werden. Sollte eine Kühlung aufgrund einer zu starken Wärmeentwicklung durch Reaktion des verbleibenden Eduktgases nötig sein, so kann dies ebenfalls im Kreislauf realisiert werden.

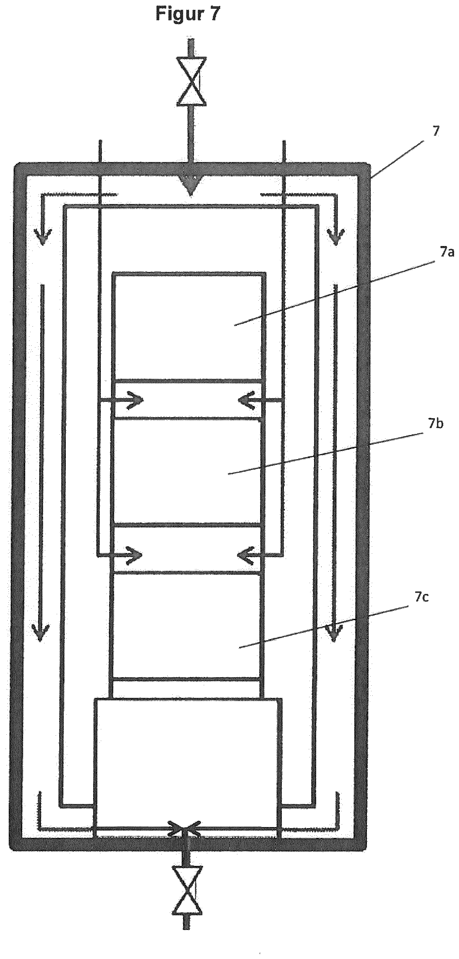

In einer bevorzugten Ausführungsform (vgl. Figuren 2 und 3) umfasst das erfindungsgemäße Verfahren im Stand-by-Betrieb das vollständige Einstellen des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases während des ersten Zeitintervalls (Stand-by-Betrieb), wobei das Gasgemisch A und/oder das Gasgemisch B und/oder das an Produktgas angereicherte Gasgemisch C und/oder das an Produktgas verarmte Gasgemisch D
- verdichtet werden, vorzugsweise zum Ausgleich von Druckverlusten; und/oder
- erwärmt werden, vorzugsweise zum Ausgleich von Wärmeverlusten; und/oder
- zumindest teilweise unter Umgehung des Katalysators, d.h. am Katalysator vorbei, geführt werden (vgl. Figur 7).

Vorzugsweise werden zum Verdichten ein Verdichter und/oder ein Ventilator eingesetzt. Verdichter, die zum Verdichten eines Gases geeignet sind, sind einem Fachmann bekannt. Je nach Beschaffenheit der Vorrichtung kann als Verdichter ein Ventilator ggf. bereits ausreichen, um die vergleichsweise geringen Druckverluste auszugleichen.

Vorzugsweise werden das Gasgemisch A und/oder das Gasgemisch B und/oder das an Produktgas angereicherte Gasgemisch C während des ersten Zeitintervalls verdichtet, um die während des ersten Zeitintervalls auftretenden Druckverluste auszugleichen.

In einer weiteren bevorzugten Ausführungsform werden das Gasgemisch A und/oder das Gasgemisch B und/oder das an Produktgas angereicherte Gasgemisch C während des ersten Zeitintervalls erwärmt, bevorzugt zum Ausgleich von Wärmeverlusten. Das Erwärmen der Gasgemische erfolgt bevorzugt mit Hilfe einer Heizung.

In einer bevorzugten Ausführungsform wird das im Kreislauf geführte Gasgemisch somit während des ersten Zeitintervalls (Stand-by-Betrieb)
- mit Hilfe einer Heizung erwärmt, um Wärmeverluste auszugleichen; und/oder
- mit einem Verdichter komprimiert, um Druckverluste auszugleichen.

Gemäß den beiden vorstehend beschriebenen besonders bevorzugten Ausführungsformen des Stand-by-Betriebs wird bevorzugt über das gesamte erste Zeitintervall ein Kreislaufstrom durch den Reaktor aufrecht gehalten. Dabei bleibt im einfachsten Fall die Strömungsführung unverändert zum normalen Betrieb, d.h. die Katalysatorbetten werden im Stand-by-Betrieb kontinuierlich durchströmt.

In einer weiteren, besonders bevorzugten Ausführungsform wird im Stand-by-Betrieb unabhängig vom Reaktortyp (Quench-Reaktor oder Reaktor mit Zwischenkühlung) die Strömungsführung innerhalb des Reaktors variiert. Durch eine entsprechende Konstruktion des Reaktors kann dazu erfindungsgemäß eine "direkte" Durchströmung realisiert werden. In diesem Fall strömt das Gasgemisch hauptsächlich durch die äußere Hülle des Reaktors, ohne mit den Katalysatorbetten in Kontakt zu kommen, was zu einem geringeren Druckverlust führt (vgl. Figur 7). Das strömende Gas in der äußeren Hülle des Reaktors unter Umgehung der Katalysatorbetten führt außerdem zu einer thermischen Isolierung der Katalysatorbetten, so dass diese nicht zu stark abkühlen.

In bevorzugten Ausführungsformen wird daher während des ersten Zeitintervalls (Stand-by-Betrieb) das im Kreislauf geführte Gasgemisch
- in einem separaten Kreislauf zum Reaktor zurückgeführt, wobei der Kreislauf mit Mitteln zum Heizen und/oder Kühlen des im Kreislauf geführten Gasgemischs ausgerüstet ist; und/oder
- in einem separaten Kreislauf zum Reaktor zurückgeführt, wobei der Kreislauf mit Mitteln zum Verdichten des im Kreislauf geführten Gasgemischs ausgerüstet ist; und/oder
- innerhalb des Reaktors zwischen dessen äußerer Hülle und dem innenliegenden Katalysatorbett bzw. den innenliegenden Katalysatorbetten geführt, so dass der Großteil des Gasgemischs (Gasgemisch B), bevorzugt das gesamte Gasgemisch, mit dem Katalysatorbett bzw. den Katalysatorbetten nicht in Kontakt kommt (vgl. Figur 7).

In einer bevorzugten Ausführungsform wird das erfindungsgemäße Verfahren während des ersten Zeitintervalls im Stand-by-Betrieb durchgeführt, und ein Wechsel zum Volllast-Betrieb während des zweiten Zeitintervalls erfolgt unter Durchlaufen eines Teillast-Betriebs während eines zwischen dem ersten Zeitintervall und dem zweiten Zeitintervall liegenden Zeitintervalls durch graduell zunehmende Bereitstellung von erstem Eduktgas in Schritt (a) und/oder zweitem Eduktgas in Schritt (b) bzw. Gasgemisch A (Frischgas) in Schritt (c).

In einer anderen bevorzugten Ausführungsform wird das erfindungsgemäße Verfahren während des ersten Zeitintervalls im Teillast-Betrieb durchgeführt, und ein Wechsel zum Volllast-Betrieb während des zweiten Zeitintervalls erfolgt durch graduell zunehmende Bereitstellung von erstem Eduktgas in Schritt (a) und/oder zweitem Eduktgas in Schritt (b) bzw. Gasgemisch A (Frischgas) in Schritt (c).

Bei den beiden vorstehend beschriebenen Ausführungsformen folgt das zweite Zeitintervall auf das erste Zeitintervall.

In einer bevorzugten Ausführungsform wird das erfindungsgemäße Verfahren während des zweiten Zeitintervalls im Volllast-Betrieb durchgeführt, und ein Wechsel zum Stand-by-Betrieb während des ersten Zeitintervalls erfolgt unter Durchlaufen eines Teillast-Betriebs während eines zwischen dem zweiten Zeitintervall und dem ersten Zeitintervall liegenden Zeitintervalls durch graduell abnehmende Bereitstellung von erstem Eduktgas in Schritt (a) und/oder zweitem Eduktgas in Schritt (b) bzw. Gasgemisch A (Frischgas) in Schritt (c).

In einer anderen bevorzugten Ausführungsform wird das erfindungsgemäße Verfahren während des zweiten Zeitintervalls im Volllast-Betrieb durchgeführt, und ein Wechsel zum Teillast-Betrieb während des ersten Zeitintervalls erfolgt durch graduell abnehmende Bereitstellung von erstem Eduktgas in Schritt (a) und/oder zweitem Eduktgas in Schritt (b) bzw. Gasgemisch A (Frischgas) in Schritt (c).

Bei den beiden vorstehend beschriebenen Ausführungsformen folgt das erste Zeitintervall auf das zweite Zeitintervall.

Bei dem erfindungsgemäßen Verfahren werden das erste und das zweite Eduktgas zu Produktgas umgesetzt.

In einer bevorzugten Ausführungsform ist das erste Eduktgas Wasserstoff. Der Wasserstoff kann aus verschiedenen Quellen stammen. Bevorzugt wird der Wasserstoff durch Elektrolyse von Wasser gewonnen. Besonders bevorzugt wird der für die Elektrolyse eingesetzte Strom dabei durch erneuerbare Energien, insbesondere durch Photovoltaik erzeugt, so dass die Verfügbarkeit des Stroms und damit auch die Verfügbarkeit des elektrolytisch gewonnenen Wasserstoffs von der Sonneneinstrahlung abhängen können.

In einer bevorzugten Ausführungsform ist das zweite Eduktgas ausgewählt aus der Gruppe bestehend aus Stickstoff, Kohlenstoffmonoxid und Kohlenstoffdioxid. Somit sind erfindungsgemäß folgende Kombinationen von erstem Eduktgas und zweitem Eduktgas bevorzugt:
(i) Wasserstoff und Stickstoff,
(ii) Wasserstoff und Kohlenstoffmonoxid, sowie
(iii) Wasserstoff und Kohlenstoffdioxid.

In einer bevorzugten Ausführungsform ist das Produktgas ausgewählt aus der Gruppe bestehend aus Ammoniak, Methanol, Ethanol, höheren Alkoholen, Aldehyden, Ketonen, Carbonsäuren und Kohlenwasserstoffen (Paraffinen und Olefinen), wobei diese ggf. auch im Gemisch vorliegen können. Ein Fachmann erkennt, aus welchen Eduktgasen diese Produktgase ggf. hergestellt werden können. Besonders bevorzugt sind das Produktgas Ammoniak, das erste Eduktgas Wasserstoff und das zweite Eduktgas Stickstoff.

In Schritt (a) des erfindungsgemäßen Verfahrens wird das erste Eduktgas bereitgestellt.

Handelt es sich bei dem ersten Eduktgas um Wasserstoff, welcher durch Elektrolyse von Wasser gewonnen wird, so erfolgt die Bereitstellung des ersten Eduktgases in Schritt (a) bevorzugt durch Entnahme des Wasserstoffs aus der Elektrolysezelle.

Alternativ kann der Wasserstoff jedoch auch aus anderen Quellen stammen, beispielsweise aus Synthesegas.

In einer bevorzugten Ausführungsform wird im Verlauf des Verfahrens der Volumenstrom des in Schritt (a) bereitgestellten ersten Eduktgases variiert, so dass in einem ersten Zeitintervall ein erster Volumenstrom und in einem zweiten Zeitintervall ein zweiter Volumenstrom des ersten Eduktgases bereitgestellt wird, welcher sich von dem ersten Volumenstrom unterscheidet.

In einer bevorzugten Ausführungsform umfasst Schritt (a) des erfindungsgemäßen Verfahrens die Teilschritte
(a₁) Erzeugen von elektrischem Strom, bevorzugt mit Hilfe regenerativer Energien, insbesondere durch Photovoltaik und/oder durch Windkraft; und
(a₂) Elektrolysieren von Wasser mit Hilfe des in Teilschritt (a₁) erzeugten elektrischen Stroms unter Erzeugung von Sauerstoff und von Wasserstoff, wobei der Wasserstoff als erstes Eduktgas bereitgestellt wird.

Bevorzugt wird dabei in Teilschritt (a₁) während des ersten Zeitintervalls weniger elektrischer Strom erzeugt und somit auch in Teilschritt (a₂) weniger Wasserstoff als erstes Eduktgas bereitgestellt als während des zweiten Zeitintervalls. In einer bevorzugten Ausführungsform wird während des ersten Zeitintervalls kein elektrischer Strom erzeugt und somit auch in Teilschritt (a₂) kein Wasserstoff als erstes Eduktgas bereitgestellt (Stand-by-Betrieb), während des zweiten Zeitintervalls hingegen schon.

In Schritt (b) des erfindungsgemäßen Verfahrens wird das zweite Eduktgas bereitgestellt.

Handelt es sich bei dem zweiten Eduktgas um Stickstoff, so erfolgt die Bereitstellung des zweiten Eduktgases in Schritt (b) bevorzugt durch Zufuhr von praktisch reinem Stickstoff, beispielsweise aus einer Luftzerlegungsanlage.

Handelt es sich bei dem zweiten Eduktgas um Kohlenstoffmonoxid, so kann dieses beispielsweise aus Synthesegas stammen, so dass in diesem Fall die Bereitstellung von Wasserstoff als erstes Eduktgas in Schritt (a) und die Bereitstellung von Kohlenstoffmonoxid als zweites Eduktgas in Schritt (b) durch die Bereitstellung von Synthesegas gleichzeitig erfolgen können.

In einer bevorzugten Ausführungsform wird im Verlauf des Verfahrens der Volumenstrom des in Schritt (b) bereitgestellten zweiten Eduktgases variiert, so dass in einem ersten Zeitintervall ein erster Volumenstrom und in einem zweiten Zeitintervall ein zweiter Volumenstrom des zweiten Eduktgases bereitgestellt wird, welcher sich von dem ersten Volumenstrom unterscheidet.

In Schritt (c) des erfindungsgemäßen Verfahrens werden das erste Eduktgas und das zweite Eduktgas unter Erhalt eines Gasgemischs A vereint, so dass das Gasgemisch A das erste Eduktgas und das zweite Eduktgas umfasst.

Zusätzlich kann das Gasgemisch A ggf. weitere Gase umfassen, beispielsweise inerte Komponenten.

Inerte Komponenten im Sinne der Erfindung sind im Hinblick auf die Synthese des Produktgases inert, d.h. bei der Umsetzung des ersten Eduktgases mit dem zweiten Eduktgas zu Produktgas werden sie nicht mit umgesetzt.

Das relative molare Verhältnis von erstem Eduktgas und zweitem Eduktgas hängt von der Stöchiometrie der Umsetzung ab, durch die das Produktgas erhalten wird.

Ist das erste Eduktgas Wasserstoff, das zweite Eduktgas Stickstoff und das Produktgas Ammoniak, so beträgt das relative molare Verhältnis von Wasserstoff zu Stickstoff bevorzugt etwa 3:1.

In einer weiteren bevorzugten Ausführungsform liegt das relative molare Verhältnis von erstem Eduktgas zu zweitem Eduktgas im Gasgemisch A im Bereich von 10:1 bis 1:10, bevorzugter 8:1 bis 1:8, noch bevorzugter 6:1 bis 1:6, am bevorzugtesten 4:1 bis 1:4, und insbesondere 2:1 bis 1:2. In einer anderen bevorzugten Ausführungsform liegt das relative molare Verhältnis von erstem Eduktgas zu zweitem Eduktgas im Gasgemisch A im Bereich von 10:1 bis 1:1, bevorzugter 8:1 bis 1:1, noch bevorzugter 6:1 bis 1:1, am bevorzugtesten 4:1 bis 1:1, und insbesondere 2:1 bis 1:1. In einer weiteren bevorzugten Ausführungsform liegt das relative molare Verhältnis von erstem Eduktgas zu zweitem Eduktgas im Gasgemisch A im Bereich von 1:1 bis 1:10, bevorzugter 1:1 bis 1:8, noch bevorzugter 1:1 bis 1:6, am bevorzugtesten 1:1 bis 1:4, und insbesondere 1:1 bis 1:2.

Bevorzugt umfasst das Gasgemisch A höchstens 30 Vol.-% Produktgas, bevorzugter höchstens 20 Vol.-%, höchstens 10, höchstens 5,0 Vol.-%, höchstens 4,0 Vol.-%, höchstens 3,0 Vol.-%, höchstens 2,0 Vol.-%, höchstens 1,0 Vol.-% oder höchstens 0,1 Vol.-% Produktgas. In einer weiteren bevorzugten Ausführungsform umfasst das Gasgemisch A kein Produktgas.

Bevorzugt beträgt der Gesamtgehalt an erstem Eduktgas und zweitem Eduktgas im Gasgemisch A mindestens 90 Vol.-%, bevorzugter mindestens 91 Vol.-%, mindestens 92 Vol.-%, mindestens 93 Vol.-%, mindestens 94 Vol.-%, mindestens 95 Vol.-%, mindestens 96 Vol.-%, mindestens 97 Vol.-%, mindestens 98 Vol.-%, oder mindestens 99 Vol.-%.

Im Unterschied zu dem Verfahren gemäß CA 2,790,545 A1 kann das erfindungsgemäße Verfahren auch bei nur geringem Gehalt inerter Komponenten im Gasgemisch A schnell und flexibel vom Volllast-Betrieb in den Teillast-Betrieb bzw. Stand-by-Betrieb versetzt werden.

Bevorzugt umfasst das Gasgemisch A höchstens 30 Vol.-% inerte Komponenten, bevorzugter höchstens 20 Vol.-%, höchstens 10, höchstens 5,0 Vol.-%, höchstens 4,0 Vol.-%, höchstens 3,0 Vol.-%, höchstens 2,0 Vol.-%, höchstens 1,0 Vol.-% oder höchstens 0,1 Vol.-% inerte Komponenten. In einer weiteren bevorzugten Ausführungsform umfasst das Gasgemisch A keine inerten Komponenten.

Die Reihenfolge der Schritte (a) und (b) des erfindungsgemäßen Verfahrens ist nicht festgelegt. Schritt (a) kann vor oder nach oder wenigstens zum Teil gleichzeitig mit Schritt (b) erfolgen. Schritt (c) des erfindungsgemäßen Verfahrens kann nach Schritten (a) und (b) oder wenigstens zum Teil gleichzeitig mit Schritt (a) und/oder (b) erfolgen. In einer besonders bevorzugten Ausführungsform erfolgen die Schritte (a) und (b) gleichzeitig und Schritt (c) im Anschluss daran.

Beispielsweise können in Schritt (a) Wasserstoff als erstes Eduktgas und in Schritt (b) Kohlenstoffmonoxid oder Kohlenstoffdioxid als zweites Eduktgas bereitgestellt werden. Sowohl der Wasserstoff als auch das Kohlenstoffmonoxid bzw. Kohlenstoffdioxid können aus Synthesegas stammen, so dass in diesem Fall gleichzeitig das erste und das zweite Eduktgas in Schritten (a) und (b) bereitgestellt und zu Gasgemisch A in Schritt (c) vereint werden können. In diesem Fall werden Schritte (a) bis (c) des erfindungsgemäßen Verfahrens nicht durch gesonderte Maßnahmen verwirklicht, sondern erfolgen gemeinsam durch die Bereitstellung von Synthesegas.

Bevorzugt werden der in Schritt (a) bereitgestellte Volumenstrom des ersten Eduktgases und/oder der in Schritt (b) bereitgestellte Volumenstrom des zweiten Eduktgases während des Verfahrens variiert, so dass
(i) während des ersten Zeitintervalls ein erster Volumenstrom und während des zweiten Zeitintervalls ein zweiter Volumenstrom des ersten Eduktgases bereitgestellt werden, wobei sich der zweite Volumenstrom von dem ersten Volumenstrom unterscheidet; und/oder
(ii) während des ersten Zeitintervalls ein erster Volumenstrom und während des zweiten Zeitintervalls ein zweiter Volumenstrom des zweiten Eduktgases bereitgestellt werden, wobei sich der zweite Volumenstrom von dem ersten Volumenstrom unterscheidet.

Bevorzugt ist der in Schritt (a) bereitgestellte Volumenstrom des ersten Eduktgases während des ersten Zeitintervalls kleiner als während des zweiten Zeitintervalls. Bevorzugt beträgt der in Schritt (a) bereitgestellte Volumenstrom des ersten Eduktgases während des ersten Zeitintervalls höchstens 99% des in Schritt (a) bereitgestellten Volumenstroms des ersten Eduktgases während des zweiten Zeitintervalls, bevorzugter höchstens 90%, höchstens 80%, höchstens 70%, höchstens 60%, höchstens 50%, höchstens 40%, höchstens 30%, höchstens 20%, höchstens 10%, höchstens 5% oder höchstens 1%.

In einer bevorzugten Ausführungsform liegt das relative Verhältnis des in Schritt (a) bereitgestellten Volumenstroms des ersten Eduktgases während des ersten Zeitintervalls zum bereitgestellten Volumenstrom des ersten Eduktgases während des zweiten Zeitintervalls im Bereich von 1:1 bis 1:100, bevorzugter 1:2 bis 1:90, noch bevorzugter 1:3 bis 1:80, 1:4 bis 1:70, 1:5 bis 1:60, 1:6 bis 1:50, 1:7 bis 1:40, 1:8 bis 1:30 oder 1:9 bis 1:20. In einer bevorzugten Ausführungsform wird während des ersten Zeitintervalls kein erstes Eduktgas bereitgestellt (Stand-by-Betrieb), während des zweiten Zeitintervalls hingegen schon.

Bevorzugt wird der in Schritt (a) bereitgestellte Volumenstrom des ersten Eduktgases während des ersten Zeitintervalls relativ zum während des zweiten Zeitintervalls bereitgestellten Volumenstrom um mindestens 5% reduziert, bevorzugter um mindestens 10%, mindestens 20%, mindestens 30%, mindestens 40%, mindestens 50%, mindestens 60%, mindestens 70%, mindestens 80%, mindestens 90% oder mindestens 99%.

Bevorzugt ist der in Schritt (b) bereitgestellte Volumenstrom des zweiten Eduktgases während des ersten Zeitintervalls kleiner als während des zweiten Zeitintervalls. Bevorzugt beträgt der in Schritt (b) bereitgestellte Volumenstrom des zweiten Eduktgases während des ersten Zeitintervalls höchstens 99% des in Schritt (b) bereitgestellten Volumenstroms des zweiten Eduktgases während des zweiten Zeitintervalls, bevorzugter höchstens 90%, höchstens 80%, höchstens 70%, höchstens 60%, höchstens 50%, höchstens 40%, höchstens 30%, höchstens 20%, höchstens 10%, höchstens 5% oder höchstens 1%.

In einer bevorzugten Ausführungsform liegt das relative Verhältnis des in Schritt (b) bereitgestellten Volumenstroms des zweiten Eduktgases während des ersten Zeitintervalls zum bereitgestellten Volumenstrom des zweiten Eduktgases während des zweiten Zeitintervalls im Bereich von 1:1 bis 1:100, bevorzugter 1:2 bis 1:90, noch bevorzugter 1:3 bis 1:80, 1:4 bis 1:70, 1:5 bis 1:60, 1:6 bis 1:50, 1:7 bis 1:40, 1:8 bis 1:30 oder 1:9 bis 1:20. In einer bevorzugten Ausführungsform wird während des ersten Zeitintervalls kein zweites Eduktgas bereitgestellt (Stand-by-Betrieb), während des zweiten Zeitintervalls hingegen schon.

Bevorzugt wird der in Schritt (b) bereitgestellte Volumenstrom des zweiten Eduktgases während des ersten Zeitintervalls relativ zum während des zweiten Zeitintervalls bereitgestellten Volumenstrom um mindestens 5% reduziert, bevorzugter um mindestens 10%, mindestens 20%, mindestens 30%, mindestens 40%, mindestens 50%, mindestens 60%, mindestens 70%, mindestens 80%, mindestens 90% oder mindestens 99%.

Als Folge der erfindungsgemäßen Variation des in Schritt (a) bereitgestellten Volumenstroms des ersten Eduktgases und/oder des in Schritt (b) bereitgestellten Volumenstroms des zweiten Eduktgases während des ersten Zeitintervalls im Vergleich zum zweiten Zeitintervall kann sich auch der jeweils bereitgestellte Volumenstrom des Gasgemischs A ändern.

Bevorzugt ist der Volumenstrom des Gasgemischs A während des ersten Zeitintervalls kleiner als während des zweiten Zeitintervalls. Bevorzugt beträgt der Volumenstrom des Gasgemischs A während des ersten Zeitintervalls höchstens 99% des Volumenstroms an Gasgemisch A während des zweiten Zeitintervalls, bevorzugter höchstens 90%, höchstens 80%, höchstens 70%, höchstens 60%, höchstens 50%, höchstens 40%, höchstens 30%, höchstens 20%, höchstens 10%, höchstens 5% oder höchstens 1%.

In Schritt (d) des erfindungsgemäßen Verfahrens wird das Gasgemisch A mit einem im Kreislauf geführten Gasgemisch unter Erhalt eines Gasgemischs B vereint, wobei das Gasgemisch B erstes Eduktgas, zweites Eduktgas, Produktgas und ggf. weitere Gase umfasst, z.B. inerte Komponenten.

Das im Kreislauf geführte Gasgemisch umfasst typischerweise nicht umgesetztes erstes Eduktgas und/oder nicht umgesetztes zweites Eduktgas und/oder Produktgas und ggf. weitere Bestandteile, insbesondere inerte Komponenten.

In einer bevorzugten Ausführungsform beträgt der Gehalt des Gasgemischs A am Gasgemisch B höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

In einer anderen bevorzugten Ausführungsform beträgt der Gehalt des Gasgemischs A am Gasgemisch B mindestens 1 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-% oder mindestens 90 Vol.-%.

In einer bevorzugten Ausführungsform beträgt der Gehalt des im Kreislauf geführten Gasgemischs am Gasgemisch B höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

In einer anderen bevorzugten Ausführungsform beträgt der Gehalt des im Kreislauf geführten Gasgemischs am Gasgemisch B mindestens 1 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-% oder mindestens 90 Vol.-%.

Bevorzugt liegt das relative Verhältnis des Volumenstroms des im Kreislauf geführten Gasgemischs zum Volumenstrom des Gasgemischs A, welche in Schritt (d) zum Gasgemisch B vereint werden, im Bereich von 10:1 bis 1:10, bevorzugter 8:1 bis 1:8, noch bevorzugter 6:1 bis 1:6, am bevorzugtesten 4:1 bis 1:4, und insbesondere 2:1 bis 1:2. In einer anderen bevorzugten Ausführungsform liegt das relative Verhältnis des Volumenstroms des im Kreislauf geführten Gasgemischs zum Volumenstrom des Gasgemischs A, welche in Schritt (d) zum Gasgemisch B vereint werden, im Bereich von 10:1 bis 1:1, bevorzugter 8:1 bis 1:1, noch bevorzugter 6:1 bis 1:1, am bevorzugtesten 4:1 bis 1:1, und insbesondere 2:1 bis 1:1. In einer weiteren bevorzugten Ausführungsform liegt das relative Verhältnis des Volumenstroms des im Kreislauf geführten Gasgemischs zum Volumenstrom des Gasgemischs A, welche in Schritt (d) zum Gasgemisch B vereint werden, im Bereich von 1:1 bis 1:10, bevorzugter 1:1 bis 1:8, noch bevorzugter 1:1 bis 1:6, am bevorzugtesten 1:1 bis 1:4, und insbesondere 1:1 bis 1:2.

Bevorzugt beträgt der Gehalt des Gasgemischs A am Gasgemisch B
- während des ersten Zeitintervalls höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%; und/oder
- während des zweiten Zeitintervalls mindestens 1 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-% oder mindestens 90 Vol.-%.

Bevorzugt beträgt der Gehalt des im Kreislauf geführten Gasgemischs am Gasgemisch B
- während des ersten Zeitintervalls mindestens 1 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-% oder mindestens 90 Vol.-%; und/oder
- während des zweiten Zeitintervalls höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

Bevorzugt beträgt das relative Verhältnis der Volumenströme des im Kreislauf geführten Gasgemischs und des Gasgemischs A, welche in Schritt (d) zum Gasgemisch B vereint werden,
- während des ersten Zeitintervalls mindestens 2:1, bevorzugter mindestens 3:1, mindestens 4:1, mindestens 5:1, mindestens 6:1, mindestens 7:1, mindestens 8:1, mindestens 9:1 oder mindestens 10:1; und/oder
- während des zweiten Zeitintervalls höchstens 10:1, bevorzugt höchstens 9:1, bevorzugt höchstens 8:1, höchstens 7:1, höchstens 6:1, höchstens 5:1, höchstens 4:1, höchstens 3:1, oder höchstens 2:1.

Die Schritte (c) und (d) können erfindungsgemäß alphabetisch nacheinander oder gleichzeitig erfolgen.

Im Schritt (e) des erfindungsgemäßen Verfahrens werden das im Gasgemisch B enthaltene erste und zweite Eduktgas an einem Katalysator zu Produktgas unter Erzeugen eines an Produktgas angereicherten Gasgemischs C umgesetzt.

Katalysatoren zur Gasphasenreaktionen sind einem Fachmann bekannt. Im Hinblick auf die erfindungsgemäß bevorzugte Synthese von Ammoniak als Produktgas wird beispielsweise verwiesen auf A. Nielsen, I. Dybkjaer, Ammonia - Catalysis and Manufacture, Springer Berlin, Kapitel 6, Seiten 202-326.

Bei dem erfindungsgemäßen Verfahren verläuft die Umsetzung in Schritt (e) bevorzugt unvollständig, so dass das an Produktgas angereicherte Gasgemisch C nicht umgesetztes erstes Eduktgas und nicht umgesetztes zweites Eduktgas umfasst.

Vorzugsweise beträgt der Gehalt der Produktgases am an Produktgas angereicherten Gasgemisch C mindestens 5 Vol.-%, bevorzugter mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-%.

In einer weiteren bevorzugten Ausführungsform liegt der Gehalt des Produktgases am an Produktgas angereicherten Gasgemisch C im Bereich von 1 bis 90 Vol.-%, bevorzugter im Bereich von 5 bis 50 Vol.-%, noch bevorzugter im Bereich von 10 bis 40 Vol.-%, am bevorzugtesten im Bereich von 15 bis 35 Vol.-%, und insbesondere im Bereich von 20 bis 30 Vol.-%.

Der Gehalt des Produktgases am an Produktgas angereicherten Gasgemisch C ist in erster Linie vom Reaktionsgleichgewicht abhängig. Bevorzugt ist der Gehalt des Produktgases am an Produktgas angereicherten Gasgemisch C unter den gegebenen Bedingungen während des ersten Zeitintervalls größer als während des zweiten Zeitintervalls, vorzugsweise mindestens doppelt so groß, bevorzugter mindestens dreimal so groß, noch bevorzugter mindestens viermal so groß, am bevorzugtesten mindestens fünfmal so groß und insbesondere mindestens sechsmal so groß.

Vorzugsweise beträgt der Gehalt des ersten Eduktgases am an Produktgas angereicherten Gasgemisch C mindestens 1 Vol.-%, bevorzugter mindestens 2 Vol.-%, mindestens 4 Vol.-%, mindestens 6 Vol.-%, mindestens 8 Vol.-%, mindestens 10 Vol.-%, mindestens 12 Vol.-%, mindestens 14 Vol.-%, mindestens 16 Vol.-%, mindestens 18 Vol.-% oder mindestens 20 Vol.-%.

Der Gehalt des ersten Eduktgases am an Produktgas angereicherten Gasgemisch C ist in erster Linie vom Reaktionsgleichgewicht abhängig. Bevorzugt ist der Gehalt des ersten Eduktgases am an Produktgas angereicherten Gasgemisch C unter den gegebenen Bedingungen während des zweiten Zeitintervalls größer als während des ersten Zeitintervalls, vorzugsweise mindestens doppelt so groß, bevorzugter mindestens dreimal so groß, noch bevorzugter mindestens viermal so groß, am bevorzugtesten mindestens fünfmal so groß und insbesondere mindestens sechsmal so groß.

Vorzugsweise beträgt der Gehalt des zweiten Eduktgases am an Produktgas angereicherten Gasgemisch C mindestens 1 Vol.-%, bevorzugter mindestens 2 Vol.-%, mindestens 4 Vol.-%, mindestens 6 Vol.-%, mindestens 8 Vol.-%, mindestens 10 Vol.-%, mindestens 12 Vol.-%, mindestens 14 Vol.-%, mindestens 16 Vol.-%, mindestens 18 Vol.-% oder mindestens 20 Vol.-%.

Der Gehalt des zweiten Eduktgases am an Produktgas angereicherten Gasgemisch C ist in erster Linie vom Reaktionsgleichgewicht abhängig. Bevorzugt ist der Gehalt des zweiten Eduktgases am an Produktgas angereicherten Gasgemisch C unter den gegebenen Bedingungen während des zweiten Zeitintervalls größer als während des ersten Zeitintervalls, vorzugsweise mindestens doppelt so groß, bevorzugter mindestens dreimal so groß, noch bevorzugter mindestens viermal so groß, am bevorzugtesten mindestens fünfmal so groß und insbesondere mindestens sechsmal so groß.

Die Synthese von Produktgas, z.B. Ammoniak, im Teillast-Betrieb ist durch eine einfache Reduzierung des Gasgemischs A (Frischgas) bzw. des ersten Eduktgases und des zweiten Eduktgases (Stickstoff und Wasserstoff im Beispiel Ammoniaksynthese) nur begrenzt möglich, da dies zu einer Erhöhung der Verweilzeit des Gasgemischs im Katalysatorbett führen kann. Die größere Verweilzeit kann wiederum höhere Umsätze insbesondere in den vorderen Katalysatorbetten bewirken. Dadurch kann sich die Temperatur innerhalb des Reaktors aufgrund der exothermen Reaktion soweit erhöhen, dass es zu Schäden am Katalysator oder auch am Reaktor kommen kann.

Um dies zu vermeiden, kann erfindungsgemäß nur der Volumenstrom eines der beiden Eduktgase (z.B. Wasserstoff) reduziert und der Volumenstrom des anderen Eduktgases (z.B. Stickstoff) konstant gehalten oder erhöht wird. Dadurch wird die Verweilzeit des Gasgemischs im Katalysatorbett nicht so stark erhöht und es tritt entsprechend keine unzulässige Temperaturerhöhung auf.

Handelt es sich bei dem zweiten Eduktgas jedoch um Stickstoff, so wird weiterhin vergleichsweise viel Energie für dessen Bereitstellung benötigt. Außerdem kann es erforderlich sein, den Spülgas-Strom (*Purge*), der ggf. für die Ausschleusung der im Stickstoff enthaltenen inerten Komponenten benötigt wird, anteilig zu erhöhen. Dies kann dann zu einem ungewünschten Verlust an erstem Eduktgas, z.B. Wasserstoff, sowie Produktgas, z.B. Ammoniak, führen.

Um ein Überhitzen im Teillast-Betrieb zu vermeiden, besteht grundsätzlich auch die Möglichkeit, den Synthesedruck zu erniedrigen, um das Gleichgewicht weiter auf die Seite der Edukte zu verschieben. Dies kann jedoch zu einer unerwünschten dynamischen mechanischen Beanspruchung des Reaktors und der sonstigen Anlageteile führen sowie die Effizienz des Prozesses verschlechtern.

In einer bevorzugten Ausführungsform wird daher die erfindungsgemäße Variation
- des in Schritt (a) bereitgestellten Volumenstroms des ersten Eduktgases und/oder
- des in Schritt (b) bereitgestellten Volumenstroms des zweiten Eduktgases und/oder
- des in Schritt (f) ausgeschleusten Volumenstroms des Produktgases kombiniert mit einer Variation der Aufteilung der (Teil-)Volumenströme des Gasgemischs B auf die einzelnen Katalysatorbetten eines Reaktors, welcher mehrere Katalysatorbetten umfasst.

In einer bevorzugten Ausführungsform des erfindungsgemäßen Verfahrens
- erfolgt die Umsetzung in Schritt (e) in mindestens einem ersten Katalysatorbett und einem zweiten Katalysatorbett;
- wobei ein erster Teilstrom des Gasgemischs B dem ersten Katalysatorbett zugeführt und das Gasgemisch, welches das erste Katalysatorbett verlässt, dem zweiten Katalysatorbett zugeführt wird;
- wobei ein zweiter Teilstrom des Gasgemischs B unter Umgehung des ersten Katalysatorbetts dem zweiten Katalysatorbett zugeführt wird;
- wobei während des ersten Zeitintervalls das relative Volumenstromverhältnis des ersten Teilstroms zum zweiten Teilstrom erhöht wird; und
- wobei während des zweiten Zeitintervalls das relative Volumenstromverhältnis des ersten Teilstroms zum zweiten Teilstrom wieder verringert wird.

Bevorzugt handelt es sich bei dem Reaktor um einen Quench-Reaktor mit mindestens zwei hintereinander geschalteten Katalysatorbetten, bevorzugter mindestens drei hintereinander geschalteten Katalysatorbetten. Ein solcher Reaktor ist beispielsweise in Figur 7 schematisch illustriert, wobei das zweite Katalysatorbett (7b) und das dritte Katalysatorbett (7c) dem ersten Katalysatorbett (7a) nachgeschaltet sind.

Zum Betreiben eines Quench-Reaktors im Teillast-Betrieb, d.h. während des ersten Zeitintervalls, werden erfindungsgemäß bevorzugt nicht nur der Gesamtvolumenstrom des Gasgemischs B, d.h. die Summe aller Teilströme auf die einzelnen Katalysatorbetten reduziert, sondern es wird bevorzugt auch das relative Verhältnis der Volumenströme auf die einzelnen Katalysatorbetten des Quench-Reaktors so variiert, dass im relativen Verhältnis während des ersten Zeitintervalls im Teillast-Betrieb mehr des Gasgemischs B auf das erste Katalysatorbett strömt als während des zweiten Zeitintervalls im Volllast-Betrieb. Dadurch wird bevorzugt trotz geringerem Durchsatz im Reaktor der Volumenstrom im ersten Katalysatorbett deutlich unterproportional reduziert. Somit steigt in diesem Katalysatorbett die Temperatur nur begrenzt an. In den weiteren, nachgeschalteten Katalysatorbetten ist der Umsatz temperatur- und partialdruckbedingt geringer, so dass auch in diesen Zonen die Temperatur trotz geringerem Quench-Volumenstrom nicht zu stark ansteigt.

Aufgrund der guten Regelbarkeit sowohl der Quench-Ströme (Gasgemisch B) als auch der Kondensationstemperatur (Ausschleusung von Produktgas) erlauben beide Konzepte eine vergleichsweise schnelle Laständerung.

Das Ziel einer solchen Strategie im Teillast-Betrieb ist es, die Temperatur in den Katalysatorbetten jeweils innerhalb des geforderten Temperaturbereichs zu halten, z.B. für eine Ammoniaksynthese bevorzugt im Bereich von 380°C bis 510°C.

In einer bevorzugten Ausführungsform werden die Teilströme des Gasgemischs B (Quench-Ströme) derart auf die Katalysatorbetten geleitet, dass die Temperatur an jedem Katalysatorbett 530°C nicht übersteigt.

Dafür wird die exotherme Synthesereaktion bevorzugt kontrolliert, typischerweise in geeignetem Ausmaß reduziert. Wird die Last reduziert, d.h. weniger Gasgemisch A (Frischgas) dem Kreislauf zugeführt und somit auch der Kreislaufstrom reduziert, steigt die Verweilzeit des Gasgemischs in allen drei Katalysatorbetten. Entsprechend würde die Reaktion in allen drei Katalysatorbetten weiter fortschreiten, als es im Volllast-Betrieb geschieht, und die maximale Temperatur von 510°C würde in allen Fällen überschritten.

Als Gegenmaßnahme wird erfindungsgemäß bevorzugt die Temperatur für das Ausschleusen des Produktgases in Schritt (f) (Kondensatortemperatur) erhöht, was zu einer Erhöhung des Stoffmengenanteils des Produktgases in der Gasphase am Austritt des Kondensators führt, und somit auch am Eintritt in den Reaktor. Folglich wird der Umsatz über die Verschiebung des Reaktionsgleichgewichtes und der Reaktionskinetik verringert.

Trotz dieser Maßnahme kann eine sinkende Last ggf. dennoch zu einer zu hohen Austrittstemperatur aus dem ersten Katalysatorbett führen. Daher wird bevorzugt zusätzlich mit sinkender Last zunehmend anteilig mehr Gasgemisch in das erste Katalysatorbett geleitet und dadurch der Verweilzeitanstieg dort begrenzt.

In einer bevorzugten Ausführungsform macht der erste Teilstrom des Gasgemischs B mindestens 10 Vol.-% des Gesamtvolumenstroms des Gasgemischs B aus, bevorzugter mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-%.

Bevorzugt macht der erste Teilstrom des Gasgemischs B
- während des ersten Zeitintervalls mindestens 10 Vol.-% des Gesamtvolumenstroms des Gasgemischs B aus, bevorzugter mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-%, und/oder
- während des zweiten Zeitintervalls mindestens 10 Vol.-% des Gesamtvolumenstroms des Gasgemischs B aus, bevorzugter mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-%.

Bevorzugt macht beim Teillast-Betrieb während des ersten Zeitintervalls der erste Teilstrom des Gasgemischs B mindestens 40 Vol.-%, bevorzugter mindestens 45 Vol.-% oder mindestens 50 Vol.-% des Gesamtvolumenstroms des Gasgemischs B aus.

Bevorzugt macht
- während des ersten Zeitintervalls (Teillast-Betrieb) der erste Teilstrom des Gasgemischs B mehr als 50 Vol.-%, bevorzugter mindestens 55 Vol.-% oder mindestens 60 Vol.-% des Gesamtvolumenstroms des Gasgemischs B und der zweite Teilstrom, oder im Falle von mehr als zwei Katalysatorbetten die Summe aller weiteren Teilströme, weniger als 50 Vol.-%, bevorzugter höchstens 45 Vol.-% oder höchstens 40 Vol.-% des Gesamtvolumenstroms des Gasgemischs B aus, und/oder
- während des zweiten Zeitintervalls (Vollast-Betrieb) der erste Teilstrom des Gasgemischs B weniger als 50 Vol.-%, bevorzugter höchstens 45 Vol.-% oder höchstens 40 Vol.-%des Gesamtvolumenstroms des Gasgemischs B und der zweite Teilstrom, oder im Falle von mehr als zwei Katalysatorbetten die Summe aller weiteren Teilströme, mehr als 50 Vol.-%, bevorzugter mindestens 55 Vol.-% oder mindestens 60 Vol.-% des Gesamtvolumenstroms des Gasgemischs B aus.

Bevorzugt macht der zweite Teilstrom des Gasgemischs B, welcher unter Umgehung des ersten Katalysatorbetts dem zweiten Katalysatorbett zugeführt wird, höchstens 99 Vol.-% des Gesamtvolumenstroms des Gasgemischs B aus, bevorzugter höchstens 90 Vol.-%, höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

Bevorzugt macht der zweite Teilstrom des Gasgemischs B, welcher unter Umgehung des ersten Katalysatorbetts dem zweiten Katalysatorbett zugeführt wird,
- während des ersten Zeitintervalls höchstens 99 Vol.-% des Gesamtvolumenstroms des Gasgemischs B aus, bevorzugter höchstens 90 Vol.-%, höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%; und/oder
- während des zweiten Zeitintervalls höchstens 99 Vol.-% des Gesamtvolumenstroms des Gasgemischs B aus, bevorzugter höchstens 90 Vol.-%, höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

In einer bevorzugten Ausführungsform liegt das relative Verhältnis des ersten Teilstroms des Gasgemischs B während des ersten Zeitintervalls zum ersten Teilstrom des Gasgemischs B während des zweiten Zeitintervalls im Bereich von 10:1 bis 1:1, bevorzugter im Bereich von 8:1 bis 1,1:1, noch bevorzugter im Bereich von 6:1 bis 1,2:1, noch bevorzugter im Bereich von 5:1 bis 1,3:1 am bevorzugtesten im Bereich von 4:1 bis 1,4:1, und insbesondere im Bereich von 3:1 bis 1,5:1.

Bevorzugt beträgt das Verhältnis des ersten Teilstroms des Gasgemischs B während des ersten Zeitintervalls zum ersten Teilstrom des Gasgemischs B während des zweiten Zeitintervalls mindestens 1,1:1, bevorzugter mindestens 1,2:1, noch bevorzugter mindestens 1,3:1, mindestens 1,4:1, mindestens 1,5:1, mindestens 1,6:1, mindestens 1,7:1, mindestens 1,8:1, mindestens 1,9:1, mindestens 2:1, mindestens 3:1, mindestens 4:1, mindestens 5:1, mindestens 6:1, mindestens 7:1, mindestens 8:1, mindestens 9:1 oder mindestens 10:1.

In einer bevorzugten Ausführungsform liegt das relative Verhältnis des zweiten Teilstroms des Gasgemischs B während des ersten Zeitintervalls zum zweiten Teilstroms des Gasgemischs B während des zweiten Zeitintervalls im Bereich von 1:90 bis 1:1, bevorzugter im Bereich von 1:70 bis 1:2, bevorzugter im Bereich von 1:60 bis 1:3, noch bevorzugter im Bereich von 1:50 bis 1:4, am bevorzugtesten im Bereich von 1:40 bis 1:5, und insbesondere im Bereich von 1:30 bis 1:6.

Bevorzugt beträgt das Verhältnis des zweiten Teilstroms des Gasgemischs B während des ersten Zeitintervalls zum zweiten Teilstrom des Gasgemischs B während des zweiten Zeitintervalls höchstens 1:90, bevorzugter höchstens 1:80, noch bevorzugter höchstens 1:70, höchstens 1:60, höchstens 1:50, höchstens 1:40, höchstens 1:30, höchstens 1:20, höchstens 1:10, höchstens 1:5, höchstens 1:4, höchstens 1:3 oder höchstens 1:2.

Bevorzugt liegt das relative Verhältnis von erstem Teilstrom des Gasgemischs B zu zweitem Teilstrom des Gasgemischs B
- während des ersten Zeitintervalls im Bereich von 99:0,1 bis 1:1, bevorzugter im Bereich von 95:1 bis 2:1, bevorzugter im Bereich von 90:2 bis 3:1, 85:3 bis 4:1, 80:4 bis 5:1, 75:4,5 bis 8:1 oder 70:5 bis 10:1; und/oder
- während des zweiten Zeitintervalls im Bereich von 1,1:1 bis 10:1, bevorzugter im Bereich von 1,2:1 bis 8:1, noch bevorzugter 1,3:1 bis 6:1, am bevorzugtesten 1,4:1 bis 4:1 und insbesondere 1,5:1 bis 2:1.

Bevorzugt beträgt das relative Verhältnis von erstem Teilstrom des Gasgemischs B zu zweitem Teilstrom des Gasgemischs B
- während des ersten Zeitintervalls mindestens 10:1, bevorzugter mindestens 20:1, mindestens 30:1, mindestens 40:1, mindestens 50:1, mindestens 60:1, mindestens 70:1, mindestens 80:1, mindestens 90:1 oder mindestens 99:1; und/oder
- während des zweiten Zeitintervalls mindestens 1,1:1, bevorzugter mindestens 1,2:1, mindestens 1,3:1, mindestens 1,4:1, mindestens 1,5:1, mindestens 1,6:1, mindestens 1,7:1, mindestens 1,8:1, mindestens 1,9:1, mindestens 2:1, mindestens 5:1 oder mindestens 10:1.

Bevorzugt wird das erfindungsgemäße Verfahren mit Hilfe mindestens eines Katalysatorbetts durchgeführt, bevorzugter mit mindestens zwei Katalysatorbetten, und noch bevorzugter mit mindestens drei Katalysatorbetten.

Bevorzugt liegt die Temperatur in den Katalysatorbetten im Bereich von 100°C bis 800°C, bevorzugter im Bereich von 200°C bis 700°C, noch bevorzugter im Bereich von 300°C bis 600°C, am bevorzugtesten im Bereich von 350°C bis 550°C und insbesondere im Bereich von 380°C bis 510°C.

In einer bevorzugten Ausführungsform beträgt die Temperatur in den Katalysatorbetten mindestens 100°C, bevorzugter mindestens 150°C, mindestens 200°C, mindestens 300°C, mindestens 350°C oder mindestens 380°C.

In einer anderen bevorzugten Ausführungsform beträgt die Temperatur in den Katalysatorbetten höchstens 800°C, bevorzugter höchstens 750°C, höchstens 700°C, höchstens 650°C, höchstens 600°C, höchstens 550°C oder höchstens 510°C.

Im Schritt (f) des erfindungsgemäßen Verfahrens wird Produktgas aus dem an Produktgas angereicherten Gasgemisch C unter Erzeugung eines an Produktgas verarmten Gasgemischs D ausgeschleust.

Bevorzugt umfasst das Ausschleusen die Abkühlung und Kondensierung des Produktgases mit Hilfe einer geeigneten Vorrichtung, insbesondere mit Hilfe eines Wärmeüberträgers mit Kühlmedium, und die anschließende Abtrennung des kondensierten Produktgases in einer geeigneten Separationsvorrichtung.

Im Hinblick auf die Abtrennung des erfindungsgemäß synthetisierten Ammoniak als Produktgas wird beispielsweise ebenfalls vollumfänglich verwiesen auf A. Nielsen, I. Dybkjaer, Ammonia - Catalysis and Manufacture, Springer Berlin, 1995, Kapitel 6, Seiten 202-326.

Schritt (f) des erfindungsgemäßen Verfahrens wird bevorzugt durch Beeinflussung der Temperatur für die Kondensierung des Produktgases variiert.

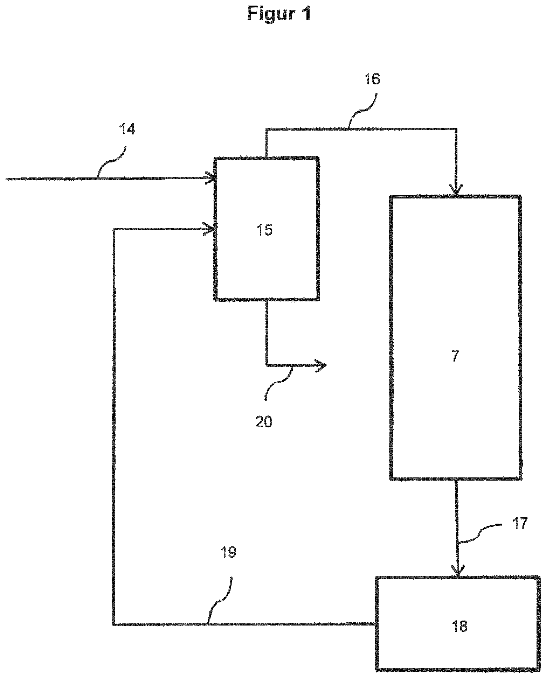

Gasphasenreaktionen wie z.B. die Ammoniaksynthese sind Gleichgewichtsreaktionen, deren Gleichgewichtslage von den Partialdrücken der Edukt- und Produktgase sowie der Temperatur abhängt. Um die beiden Eduktgase, z.B. Stickstoff und Wasserstoff, möglichst vollständig abreagieren zu lassen, wird im Synthesekreislauf bevorzugt kontinuierlich Produktgas, z.B. Ammoniak ausgeschleust, bevorzugt auskondensiert, und die verbleibende Gasphase in einem Kreislauf zurückgeführt (vgl. Figur 1). Das bedeutet, dass der Reaktion kontinuierlich Produktgas entzogen wird, so dass laut Massenwirkungsgesetz die Eduktgase weiter reagieren können, um sich dem chemischen Gleichgewicht anzunähern. Die verbleibende Restmenge an Produktgas im Gasgemisch ist neben dem Druck von der Kondensatoraustrittstemperatur abhängig.

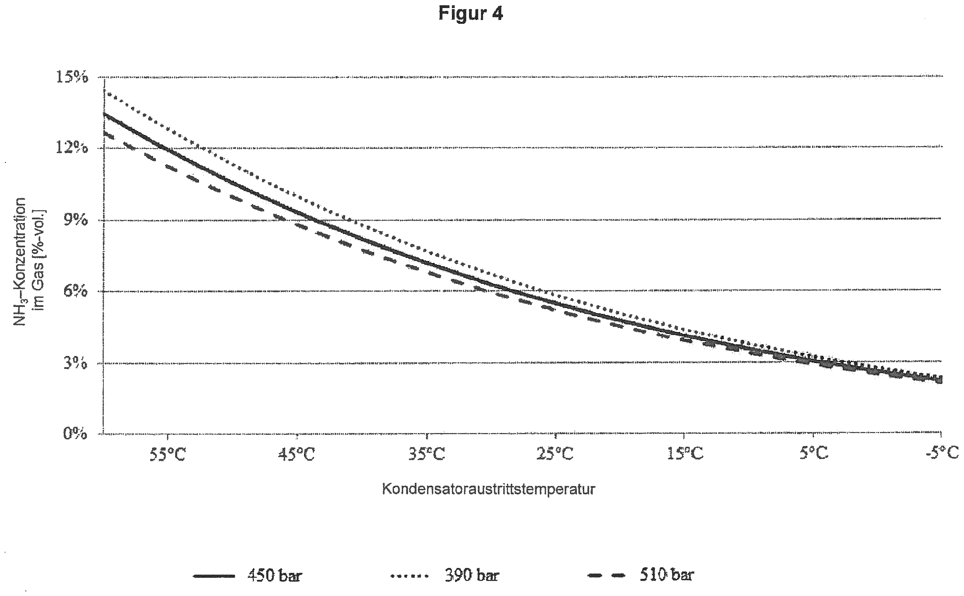

Zum Teillast-Betrieb wird die Kondensatoraustrittstemperatur bevorzugt erhöht. Dies führt zu einem höheren Partialdruck des Produktgases im Gleichgewicht mit der auskondensierten Flüssigkeit und damit zu einem höheren Anteil an Produktgas im Gasgemisch. Dadurch wird die Gleichgewichtslage in den Katalysatorbetten auf die Seite des Produktgases, z.B. Ammoniak verschoben und es wird entsprechend ein geringerer Umsatz erzielt sowie weniger Reaktionswärme freigesetzt, so dass ein Überhitzen des Reaktors bei geeignet gewählter Betriebstemperatur des Kondensators vermieden werden kann (vgl. Figur 4).

In einer bevorzugten Ausführungsform werden aus dem an Produktgas angereicherten Gasgemisch C mindestens 1 Vol.-%, bevorzugter mindestens 10 Vol.-% mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-% des Produktgases ausgeschleust, bezogen auf die Gesamtmenge des Produktgases, welches vor der Ausschleusung in dem an Produktgas angereicherten Gasgemisch C enthalten ist.

In einer anderen bevorzugten Ausführungsform werden aus dem an Produktgas angereicherten Gasgemisch C höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-% oder höchstens 1 Vol.-% des Produktgases ausgeschleust, bezogen auf die Gesamtmenge des Produktgases, welches vor der Ausschleusung in dem an Produktgas angereicherten Gasgemisch C enthalten ist.

In einer bevorzugten Ausführungsform liegt das relative Verhältnis des Gehalts an Produktgas am Gasgemisch C zum Gehalt an Produktgas am Gasgemisch D im Bereich von 10:1 bis 1:1, bevorzugter 8:1 bis 1:1, noch bevorzugter 6:1 bis 1:1, am bevorzugtesten 4:1 bis 1:1, und insbesondere 2:1 bis 1:1.

In einer bevorzugten Ausführungsform wird im Verlauf des Verfahrens der Volumenstrom des in Schritt (f) ausgeschleusten Produktgases variiert, so dass in einem ersten Zeitintervall ein erster Volumenstrom und in einem zweiten Zeitintervall ein zweiter Volumenstrom des Produktgases ausgeschleust wird, welcher sich von dem ersten Volumenstrom unterscheidet.

Bevorzugt ist der in Schritt (f) ausgeschleuste Volumenstrom des Produktgases während des zweiten Zeitintervalls größer als während des ersten Zeitintervalls, vorzugsweise mindestens doppelt so groß, bevorzugter mindestens dreimal so groß, noch bevorzugter mindestens viermal so groß, am bevorzugtesten mindestens fünfmal so groß und insbesondere mindestens sechsmal so groß. In einer bevorzugten Ausführungsform (Stand-by-Betrieb) wird während des ersten Zeitintervalls nahezu kein oder gar kein Produktgas ausgeschleust, während des zweiten Zeitintervalls hingegen schon.

In einer bevorzugten Ausführungsform liegt das relative Verhältnis des in Schritt (f) ausgeschleusten Volumenstroms an Produktgas während des ersten Zeitintervalls zum ausgeschleusten Volumenstrom des Produktgases während des zweiten Zeitintervalls im Bereich von 1:1 bis 1:100, bevorzugter 1:2 bis 1:90, noch bevorzugter 1:3 bis 1:80, 1:4 bis 1:70, 1:5 bis 1:60, 1:6 bis 1:50, 1:7 bis 1:40, 1:8 bis 1:30 oder 1:9 bis 1:20.

Bevorzugt wird der in Schritt (f) ausgeschleuste Volumenstrom des Produktgases während des ersten Zeitintervalls relativ zum während des zweiten Zeitintervalls ausgeschleusten Volumenstrom um mindestens 5% reduziert, bevorzugter um mindestens 10%, mindestens 20%, mindestens 30%, mindestens 40%, mindestens 50%, mindestens 60%, mindestens 70%, mindestens 80%, mindestens 90% oder mindestens 99%.

In einer bevorzugten Ausführungsform wird während des ersten Zeitintervalls kein Produktgas aus dem Gasgemisch C ausgeschleust, so dass das Gasgemisch D, welches zurückgeführt wird, die gesamte Menge des Produktgases enthält (Stand-by-Betrieb).

In einer bevorzugten Ausführungsform beträgt der Gehalt an Produktgas im an Produktgas verarmten Gasgemisch D mindesten 0,5 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-% oder mindestens 99 Vol.-%.

In einer anderen bevorzugten Ausführungsform beträgt der Gehalt an Produktgas im an Produktgas verarmten Gasgemisch D höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-% oder höchstens 1 Vol.-%.

Der Gehalt an Produktgas im an Produktgas verarmten Gasgemisch D ist vom Phasengleichgewicht/Partialdruck abhängig. Bevorzugt ist der Gehalt an Produktgas im an Produktgas verarmten Gasgemisch D unter den gegebenen Bedingungen während des zweiten Zeitintervalls größer als während des ersten Zeitintervalls, vorzugsweise mindestens doppelt so groß, bevorzugter mindestens dreimal so groß, noch bevorzugter mindestens viermal so groß, am bevorzugtesten mindestens fünfmal so groß und insbesondere mindestens sechsmal so groß. In einer bevorzugten Ausführungsform (Stand-by-Betrieb) wird während des ersten Zeitintervalls nahezu kein oder gar kein Produktgas ausgeschleust, während des zweiten Zeitintervalls hingegen schon.

Bevorzugt beträgt der Gehalt an Produktgas im an Produktgas verarmten Gasgemisch D
- während des ersten Zeitintervalls mindestens 1 Vol.-%, bevorzugter mindestens 5 Vol.-%, mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-% oder mindestens 90 Vol.-%; und/oder
- während des zweiten Zeitintervalls höchstens 90 Vol.-%, bevorzugter höchstens 80 Vol.-%, höchstens 70 Vol.-%, höchstens 60 Vol.-%, höchstens 50 Vol.-%, höchstens 40 Vol.-%, höchstens 30 Vol.-%, höchstens 20 Vol.-%, höchstens 10 Vol.-%, höchstens 5 Vol.-% oder höchstens 1 Vol.-%.

Im Schritt (g) des erfindungsgemäßen Verfahrens wird zumindest ein Teil des an Produktgas verarmten Gasgemischs D als im Kreislauf geführtes Gasgemisch zu Schritt (d) rückgeführt.

Bevorzugt beträgt der relative Volumenanteil des Gasgemischs D, das zu Schritt (d) rückgeführt wird, mindestens 1 Vol.-%, bevorzugter mindestens 10 Vol.-%, mindestens 20 Vol.-%, mindestens 30 Vol.-%, mindestens 40 Vol.-%, mindestens 50 Vol.-%, mindestens 60 Vol.-%, mindestens 70 Vol.-%, mindestens 80 Vol.-%, mindestens 90 Vol.-%, mindestens 95 Vol.-% oder mindestens 99 Vol.-%, jeweils bezogen auf das Gesamtvolumen des an Produktgas verarmten Gasgemischs D.

Bevorzugt beträgt der relative Volumenanteil des Gasgemischs D, das zu Schritt (d) rückgeführt wird, höchstens 99%, bevorzugter höchstens 90%, höchstens 80%, höchstens 70%, höchstens 60%, höchstens 50%, höchstens 40%, höchstens 30%, höchstens 20%, höchstens 10% oder höchstens 1%, jeweils bezogen auf das Gesamtvolumen des an Produktgas verarmten Gasgemischs D.

In einer bevorzugten Ausführungsform wird im Verlauf des Verfahrens der Volumenstrom des an Produktgas verarmten Gasgemischs D, das in Schritt (g) als im Kreislauf geführtes Gasgemisch zu Schritt (d) rückgeführt wird, variiert, so dass in einem ersten Zeitintervall ein erster Volumenstrom und in einem zweiten Zeitintervall ein zweiter Volumenstrom des an Produktgas verarmten Gasgemischs D zu Schritt (d) rückgeführt wird, welcher sich von dem ersten Volumenstrom unterscheidet.

Bevorzugt liegt das Verhältnis des Volumenstroms des an Produktgas verarmten Gasgemischs D, der während des ersten Zeitintervalls zu Schritt (d) rückgeführt wird, zu dem Volumenstrom des an Produktgas verarmten Gasgemischs D, der während des zweiten Zeitintervalls zu Schritt (d) rückgeführt wird, im Bereich von 10:1 bis 1:10, bevorzugter 8:1 bis 1:8, noch bevorzugter 6:1 bis 1:6, am bevorzugtesten 4:1 bis 1:4, und insbesondere 2:1 bis 1:2. In einer anderen bevorzugten Ausführungsform liegt das Verhältnis des Volumenstroms des an Produktgas verarmten Gasgemisch D, der während des ersten Zeitintervalls zu Schritt (d) rückgeführt wird, zu dem Volumenstrom des an Produktgas verarmten Gasgemischs D, der während des zweiten Zeitintervalls zu Schritt (d) rückgeführt wird, im Bereich von 10:1 bis 1:1, bevorzugter 8:1 bis 1:1, noch bevorzugter 6:1 bis 1:1, am bevorzugtesten 4:1 bis 1:1, und insbesondere 2:1 bis 1:1. In einer weiteren bevorzugten Ausführungsform liegt das Verhältnis des Volumenstroms des an Produktgas verarmten Gasgemisch D, der während des ersten Zeitintervalls zu Schritt (d) rückgeführt wird, zu dem Volumenstrom des an Produktgas verarmten Gasgemischs D, der während des zweiten Zeitintervalls zu Schritt (d) rückgeführt wird, im Bereich von 1:1 bis 1:10, bevorzugter 1:1 bis 1:8, noch bevorzugter 1:1 bis 1:6, am bevorzugtesten 1:1 bis 1:4, und insbesondere 1:1 bis 1:2.

Die Schritte (a) bis (g) des erfindungsgemäßen Verfahrens werden bevorzugt in alphabetischer Reihenfolge durchgeführt, können aber auch in davon abweichender Reihenfolge durchgeführt werden. Dabei können einzelne Schritte des erfindungsgemäßen Verfahrens teilweise oder vollständig zeitgleich mit anderen Schritten des erfindungsgemäßen Verfahrens durchgeführt werden.

Bevorzugt umfasst das erfindungsgemäße Verfahren das Beeinflussen der pro Zeiteinheit gebildeten Menge des Produktgases durch die Variation des in Schritt (a) bereitgestellten Volumenstroms des ersten Eduktgases und/oder die Variation des in Schritt (b) bereitgestellten Volumenstroms des zweiten Eduktgases und/oder die Variation des in Schritt (f) ausgeschleusten Volumenstroms des Produktgases.

Bevorzugt erfolgt dabei die Variation der bereitgestellten Volumenströme an erstem und/oder zweitem Eduktgas und/oder die Variation des ausgeschleusten Volumenstroms des Produktgases in Abhängigkeit der Verfügbarkeit von elektrischem Strom, noch bevorzugter in Abhängigkeit der Verfügbarkeit von Strom, welcher durch erneuerbare Energien, insbesondere Photovoltaik und/oder Windkraft, erzeugt wird. Noch bevorzugter erfolgt die Variation in Abhängigkeit der Verfügbarkeit von erstem und/oder zweitem Eduktgas, bevorzugt Wasserstoff, welcher bevorzugt aus der Elektrolyse von Wasser gewonnen wird.

Die "pro Zeiteinheit gebildete Menge" im Sinne der Erfindung bezeichnet bevorzugt den Umsatz des im Verfahren eingesetzten Wasserstoffs zu Produktgas.

Im Hinblick auf die pro Zeiteinheit gebildete Menge an Produktgas verhalten sich die Zustände des erfindungsgemäßen Verfahrens gemäß folgender Rangfolge: Stand-by-Betrieb < Teillast-Betrieb < Volllast-Betrieb.

Bevorzugt beträgt die pro Zeiteinheit gebildete Menge an Produktgas während des Teillast-Betriebs relativ höchstens 90% der pro Zeiteinheit gebildeten Menge an Produktgas während des Volllast-Betriebs, bevorzugter höchstens 80%, noch bevorzugter höchstens 70%, oder höchstens 60% oder höchstens 50%, am bevorzugtesten höchstens 40% oder höchstens 30% und insbesondere höchstens 20% oder höchstens 10% der pro Zeiteinheit gebildeten Menge an Produktgas während des Volllast-Betriebs.

Bevorzugt beträgt die pro Zeiteinheit gebildete Menge an Produktgas während des Stand-by-Betriebs praktisch 0%.

Ein weiterer Aspekt der Erfindung betrifft eine Vorrichtung zur Herstellung eines Produktgases aus einem ersten Eduktgas und einem zweiten Eduktgas, insbesondere gemäß dem Verfahren nach einem der Ansprüche 1 bis 12, umfassend die folgenden miteinander in Wirkverbindung stehenden Komponenten:
- eine erste Einlassvorrichtung konfiguriert zur Bereitstellung des ersten Eduktgases;
- eine zweite Einlassvorrichtung konfiguriert zur Bereitstellung des zweiten Eduktgases;
- einen Reaktor konfiguriert zur Synthese eines Produktgases aus dem ersten Eduktgas und dem zweiten Eduktgas, zur Erzeugung eines das Produktgas angereicherten Gasgemischs C unter heterogener Gaskatalyse;
- eine Ausschleusevorrichtung konfiguriert zum Ausschleusen von Produktgas aus dem an Produktgas angereicherten an Produktgas angereicherten Gasgemisch C unter Erzeugung eines an Produktgas verarmten Gasgemischs D; und
- eine Steuerungsvorrichtung, welche konfiguriert ist, die Vorrichtung in einen ersten Betriebszustand oder in einen zweiten Betriebszustand zu versetzen, wobei sich der erste Betriebszustand von dem zweiten Betriebszustand
   (i) in einem geringeren Volumenstrom des ersten Eduktgases, welches durch die erste Einlassvorrichtung bereitgestellt wird, und/oder in einem geringeren Volumenstrom des zweiten Eduktgases, welches durch die zweite Einlassvorrichtung bereitgestellt wird, und/oder in einem geringeren Volumenstrom des Produktgases, welches durch die Ausschleusevorrichtung ausgeschleust wird,
      und/oder
   (ii) in einer geringeren pro Zeiteinheit gebildeten Menge des Produktgases unterscheidet,
wobei der Reaktor (7) mindestens ein Katalysatorbett umfasst, welches einen radialen Abstand zu einer äußeren Hülle des Reaktors aufweist, wodurch sich ein durchströmbarer Freiraum zwischen dem Katalysatorbett und der äußeren Hülle des Reaktors ergibt, derart, dass in dem ersten Betriebszustand ein in den Reaktor (7) eintretendes Gasgemisch den Reaktor im Wesentlichen axial durchströmen kann, ohne das Katalysatorbett zu durchströmen, wobei
der Reaktor (7) mindestens zwei in axialer Richtung hintereinander angeordnete Katalysatorbetten (7a, 7b, 7c) umfasst, wobei Mittel vorgesehen sind, mittels derer mindestens ein Teilstrom des eintretenden Gasgemisches unter Umgehung eines ersten Katalysatorbettes (7a) unmittelbar in ein diesem ersten Katalysatorbett (7a) nachgeschaltetes zweites Katalysatorbett (7b) einströmen kann.

Alle bevorzugten Ausführungsformen, welche vorstehend im Zusammenhang mit dem erfindungsgemäßen Verfahren zur Synthese eines Produktgases bzw. zum Speichern von Energie beschrieben wurden, geltend entsprechend analog auch für die erfindungsgemäße Vorrichtung.

Bevorzugt umfasst die erfindungsgemäße Vorrichtung einen Speicher für erstes Eduktgas, vorzugsweise Wasserstoff, dessen Kapazität bevorzugt dazu ausreicht, kleinere temporäre Schwankungen bei der Verfügbarkeit des ersten Eduktgases auszugleichen.

Bevorzugt umfasst die erfindungsgemäße Vorrichtung eine Leitung, welche konfiguriert ist, ein Gasgemisch von einem Auslass des Reaktors zu einem Einlass des Reaktors im Kreislauf zu führen, wobei der Kreislauf eine Heizvorrichtung umfasst, und wobei die Steuerungsvorrichtung so konfiguriert ist, dass im ersten Betriebszustand zumindest ein Teil des den Reaktor verlassenden Gasgemischs C von dem Auslass des Reaktors über die Leitung bis zu dem Einlass des Reaktors geführt und dabei in der Heizvorrichtung erwärmt wird.

Die erfindungsgemäße Vorrichtung eignet sich besonders zur Durchführung des erfindungsgemäßen Verfahrens.

Ein weiterer Aspekt der Erfindung betrifft daher die Verwendung der erfindungsgemäßen Vorrichtung zur Synthese von Produktgas durch das erfindungsgemäße Verfahren zur Synthese von Produktgas.

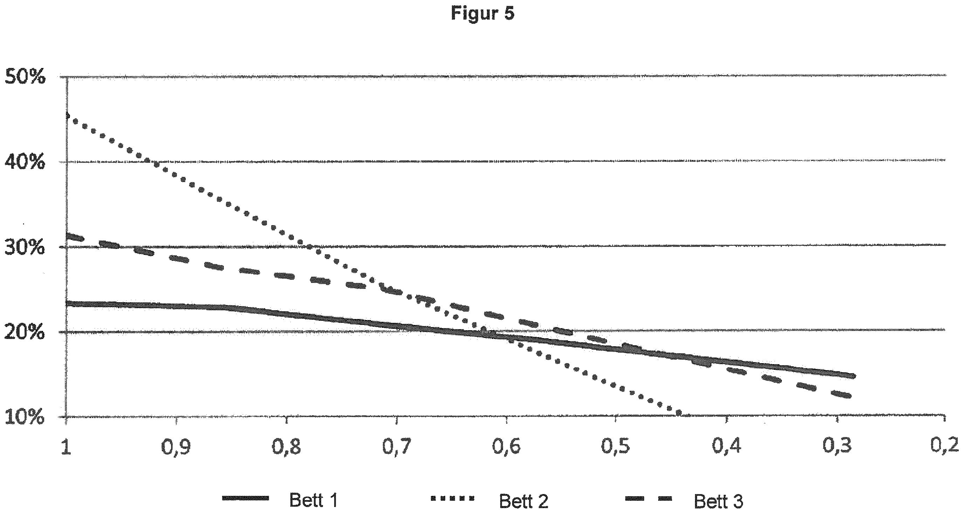

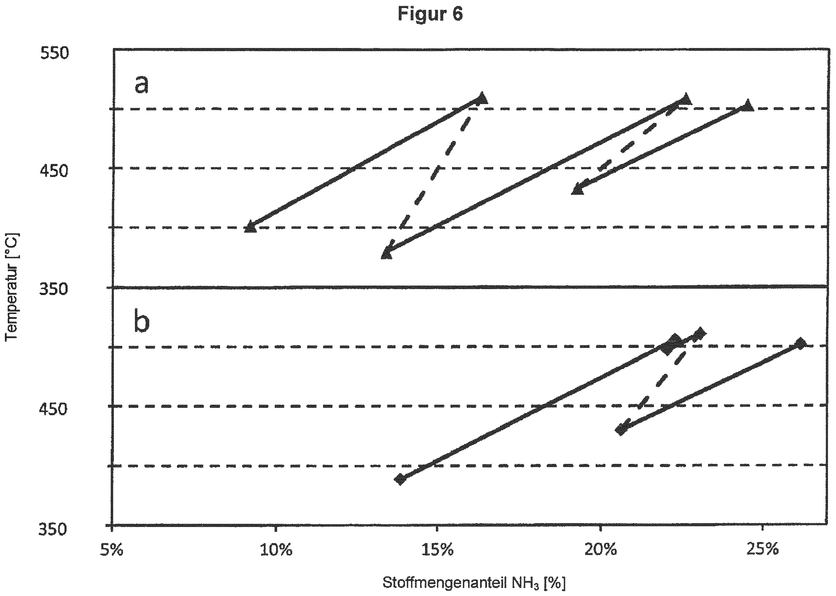

Die Erfindung wird nachfolgend anhand der Figuren 1 bis 7 näher erläutert. Die Figuren dienen jedoch lediglich der Erläuterung bevorzugter Ausführungsformen der Erfindung und sind nicht einschränkend auszulegen.

Figur 1 zeigt einen Kreislaufprozess, welcher besonders für eine Ammoniak- und Methanol-Synthese geeignet ist, aber auch für die Herstellung von Ethanol und von höheren Alkoholen, Aldehyden, Ketonen, Carbonsäuren sowie Paraffinen und Olefinen. Der Kreislauf umfasst wenigstens einen Reaktor (7), in dem ein Gasgemisch B (16) zu Produktgas (17) unter Erzeugen eines an Produktgas angereicherten Gasgemischs C umgesetzt wird. Das Produktgas (17) strömt durch einen Wärmeüberträger (18), z.B. einen Kondensator (18). Hierin wird Produktgas (19) in Abhängigkeit von der Temperatur des Wärmeüberträgers und des Drucks auskondensiert. Das nicht auskondensierte an Produktgas verarmte Gasgemisch D (16) und das kondensierte Produktgas (19) können als Kreislaufgas weiter zu einem Abscheider (15) geleitet werden, wobei es vorteilhaft ist, frisches Gasgemisch A (Frischgas) (14) mit dem im Kreislauf geführten Gasgemisch (19) zusammen in den Abscheider (15) einzuleiten, um das kondensierte Produktgas (20) in flüssiger Form besser abscheiden zu können und aus dem Prozess auszuschleusen. Das durch den Abscheider (15) nicht abgeschiedene an Produktgas verarmte Gasgemisch D (16) wird wieder zum Reaktor (7) zurückgeführt.

Der in Figur 2 dargestellte Kreislauf ist eine mögliche Variante zum Warmhalten des Kreislaufprozess im Stand-by-Betrieb. Der Reaktor (7) wird mittels des durch einen Wärmeüberträger (18) und eine Heizung (13) erwärmten Gasgemischs warmgehalten. Hierbei umfasst der Kreislauf einen Reaktor (7), einen Wärmeüberträger (18), einen Kreislaufverdichter (6) und eine Heizung (13).

Figur 3 zeigt einen kleinen, separaten Kreislauf für den Stand-by-Betrieb, wobei ein Wärmeüberträger bzw. eine Heizung (13) und ein Gebläse (21) um einen Reaktor (7) im Kreislauf zum Ausgleichen der Wärmeverluste und der Druckverluste vorgesehen sind. Das Gasgemisch gelangt über einen Einlass (12) in den Reaktor (7), den das Produktgasgemisch über den Auslass (11) verlässt, wobei über die Ausschleusevorrichtung (8), beispielsweise ein Ventil, ein Anteil des Produktgases aus dem Kreislauf ausgeschleust werden kann. Im Übrigen

## Patentansprüche

1. Ein Verfahren zur Synthese eines Produktgases umfassend die Schritte:
(a) Bereitstellen eines ersten Eduktgases;
(b) Bereitstellen eines zweiten Eduktgases;
(c) Vereinen des ersten Eduktgases und des zweiten Eduktgases unter Erhalt eines Gasgemischs A umfassend erstes Eduktgas und zweites Eduktgas;
(d) Vereinen des Gasgemischs A mit einem im Kreislauf geführten Gasgemisch unter Erhalt eines Gasgemischs B umfassend erstes Eduktgas, zweites Eduktgas und Produktgas;
(e) Umsetzen von in Gasgemisch B enthaltenem ersten Eduktgas und zweiten Eduktgas an einem Katalysator zu Produktgas unter Erzeugen eines an Produktgas angereicherten Gasgemischs C;
(f) Ausschleusen von Produktgas aus dem an Produktgas angereicherten Gasgemisch C unter Erzeugung eines an Produktgas verarmten Gasgemischs D; und
(g) Rückführen zumindest eines Teils des an Produktgas verarmten Gasgemischs D als im Kreislauf geführtes Gasgemisch zu Schritt (d);
wobei der in Schritt (a) bereitgestellte Volumenstrom des ersten Eduktgases und/oder der in Schritt (b) bereitgestellte Volumenstrom des zweiten Eduktgases und/oder der in Schritt (f) ausgeschleuste Volumenstrom des Produktgases während des Verfahrens variiert wird, weiterhin umfassend
- das vorübergehende Vermindern der pro Zeiteinheit gebildeten Menge des Produktgases während eines ersten Zeitintervalls durch Verringern des in Schritt (a) bereitgestellten Volmenstroms des ersten Eduktgases und/oder des in Schritt (b) bereitgestellten Volumenstroms des zweiten Eduktgases und/oder des in Schritt (f) ausgeschleusten Volumenstroms des Produktgases; und
- das Erhöhen der pro Zeiteinheit gebildeten Menge des Produktgases während eines zweiten Zeitintervalls durch Erhöhen des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases,
**dadurch gekennzeichnet, dass**
- die Umsetzung in Schritt (e) in mindestens einem ersten Katalysatorbett und einem zweiten Katalysatorbett erfolgt;
- ein erster Teilstrom des Gasgemischs B dem ersten Katalysatorbett zugeführt und das Gasgemisch, welches das erste Katalysatorbett verlässt, dem zweiten Katalysatorbett zugeführt wird;
- ein zweiter Teilstrom des Gasgemischs B unter Umgehung des ersten Katalysatorbetts dem zweiten Katalysatorbett zugeführt wird;
- während des ersten Zeitintervalls das relative Volumenstromverhältnis des ersten Teilstroms zum zweiten Teilstrom erhöht wird; und
- während des zweiten Zeitintervalls, das relative Volumenstromverhältnis des ersten Teilstroms zum zweiten Teilstrom wieder verringert wird.

2. Das Verfahren nach Anspruch 1, wobei der erste Teilstrom des Gasgemischs B mindestens 40 Vol.-% der Gesamtmenge des Gasgemischs B ausmacht.

3. Das Verfahren nach einem der Ansprüche 1 oder 2 umfassend das vollständige Einstellen des in Schritt (a) bereitgestellten Volumenstroms des ersten Eduktgases und/oder des in Schritt (b) bereitgestellten Volumenstroms des zweiten Eduktgases und/oder des in Schritt (f) ausgeschleusten Volumenstroms des Produktgases während mindestens eines dritten Zeitintervalls.

4. Das Verfahren nach Anspruch 3, wobei das Gasgemisch A und/oder das Gasgemisch B und/oder das an Produktgas angereicherte Gasgemisch C und/oder das an Produktgas verarmte Gasgemisch D
- verdichtet werden; und/oder
- erwärmt werden; und/oder
- wobei das Gasgemisch A und/oder das an Produktgas verarmte Gasgemisch D zumindest teilweise unter Umgehung des Katalysators geführt werden.

5. Das Verfahren nach einem der vorstehenden Ansprüche, wobei das erste Eduktgas Wasserstoff ist.

6. Das Verfahren nach Anspruch 5, wobei der Wasserstoff durch Elektrolyse von Wasser gewonnen wird.

7. Das Verfahren nach einem der vorstehenden Ansprüche, wobei die Umsetzung in Schritt (e) unvollständig verläuft, so dass das an Produktgas angereicherte Gasgemisch C nicht umgesetztes erstes Eduktgas und nicht umgesetztes zweites Eduktgas umfasst.

8. Das Verfahren nach einem der vorstehenden Ansprüche, wobei der Gehalt an Produktgas in an Produktgas verarmten Gasgemisch D mindestens 0,5 Vol.-% beträgt.

9. Das Verfahren nach einem der vorstehenden Ansprüche, wobei das zweite Eduktgas Stickstoff ist und das Produktgas Ammoniak ist.

10. Das Verfahren nach einem der Ansprüche 1 bis 8, wobei das zweite Eduktgas Kohlenstoffmonoxid oder Kohlenstoffdioxid ist.

11. Das Verfahren nach einem der vorstehenden Ansprüche, wobei das Gasgemisch A kein Produktgas umfasst.

12. Das Verfahren nach einem der Ansprüche 1 bis 11, wobei das Ausschleusen des Produktgases in Schritt (f) durch Auskondensieren mittels eines Kondensators erfolgt und im ersten Zeitintervall die Austrittstemperatur des Kondensators erhöht wird.

13. Eine Anlage zur Herstellung eines Produktgases aus einem ersten Eduktgas und einem zweiten Eduktgas, insbesondere gemäß dem Verfahren nach einem der vorigen Ansprüche 1 bis 12, umfassend die folgenden miteinander in Wirkverbindung stehenden Komponenten:
- eine erste Einlassvorrichtung konfiguriert zur Bereitstellung des ersten Eduktgases;
- eine zweite Einlassvorrichtung konfiguriert zur Bereitstellung des zweiten Eduktgases;
- einen Reaktor (7) konfiguriert zur Synthese eines Produktgases aus dem ersten Eduktgas und dem zweiten Eduktgas, zur Erzeugung eines an Produktgas angereicherten Gasgemischs C unter heterogener Gaskatalyse;
- eine Ausschleusevorrichtung (8) konfiguriert zum Ausschleusen von Produktgas aus dem an Produktgas angereicherten an Produktgas angereicherten Gasgemisch C unter Erzeugung eines an Produktgas verarmten Gasgemischs D; und
- eine Steuerungsvorrichtung, welche konfiguriert ist, die Vorrichtung in einen ersten Betriebszustand oder in einen zweiten Betriebszustand zu versetzen, wobei sich der erste Betriebszustand von dem zweiten Betriebszustand
(i) in einem geringeren Volumenstrom des ersten Eduktgases, welches durch die erste Einlassvorrichtung bereitgestellt wird, und/oder in einem geringeren Volumenstrom des zweiten Eduktgases, welches durch die zweite Einlassvorrichtung bereitgestellt wird, und/oder in einem geringeren Volumenstrom des Produktgases, welches durch die Ausschleusevorrichtung (8) ausgeschleust wird,
und/oder
(ii) in einer geringeren pro Zeiteinheit gebildeten Menge des Produktgases unterscheidet,
wobei der Reaktor (7) mindestens ein Katalysatorbett umfasst, welches einen radialen Abstand zu einer äußeren Hülle des Reaktors aufweist, wodurch sich ein durchströmbarer Freiraum zwischen dem Katalysatorbett und der äußeren Hülle des Reaktors ergibt, derart, dass in dem ersten Betriebszustand ein in den Reaktor (7) eintretendes Gasgemisch den Reaktor im Wesentlichen axial durchströmen kann, ohne das Katalysatorbett zu durchströmen, wobei
der Reaktor (7) mindestens zwei in axialer Richtung hintereinander angeordnete Katalysatorbetten (7a, 7b, 7c) umfasst, wobei Mittel vorgesehen sind, mittels derer mindestens ein Teilstrom des eintretenden Gasgemisches unter Umgehung eines ersten Katalysatorbettes (7a) unmittelbar in ein diesem ersten Katalysatorbett (7a) nachgeschaltetes zweites Katalysatorbett (7b) einströmen kann.

14. Anlage nach dem vorhergehenden Anspruch, welche eine Leitung (10) umfasst, welche konfiguriert ist, ein Gasgemisch von einem Auslass (11) des Reaktors (7) zu einem Einlass (12) des Reaktors (7) im Kreislauf zu führen, wobei der Kreislauf eine Heizvorrichtung (13) umfasst, und wobei die Steuerungsvorrichtung so konfiguriert ist, dass im ersten Betriebszustand zumindest ein Teil des den Reaktor (7) verlassenden Gasgemischs C von dem Auslass (11) des Reaktors (7) über die Leitung (10) bis zu dem Einlass (12) des Reaktors (7) geführt und dabei in der Heizvorrichtung (13) erwärmt wird.

15. Verwendung einer Anlage nach einem der Ansprüche 13 oder 14 in einem Verfahren nach einem der vorhergehenden Verfahrensansprüche.

## Claims

1. Process for synthesizing a product gas, comprising the steps:
(a) provision of a first feed gas;
(b) provision of a second feed gas;
(c) combining of the first feed gas and the second feed gas to give a gas mixture A comprising first feed gas and second feed gas;
(d) combining of the gas mixture A with a circulated gas mixture to give a gas mixture B comprising first feed gas, second feed gas and product gas;
(e) reaction of the first feed gas and second feed gas present in gas mixture B over a catalyst to a product gas with production of a gas mixture C enriched in product gas;
(f) removal of product gas from the gas mixture C enriched in product gas with production of a gas mixture D depleted in product gas; and
(g) recirculation of at least part of the gas mixture D depleted in product gas as circulated gas mixture to step (d);
where the volume flow of the first feed gas provided in step (a) and/or the volume flow of the second feed gas provided in step (b) and/or the volume flow of the product gas removed in step (f) is varied during the process, further comprising
- the temporary reduction of the amount of product gas formed per unit time during a first time interval by reduction of the volume flow of the first feed gas provided in step (a) and/or the volume flow of the second feed gas provided in step (b) and/or the volume flow of the product gas removed in step (f); and
- increasing of the amount of product gas formed per unit time during a second time interval by increasing the volume flow of the first feed gas provided in step (a) and/or the volume flow of the second feed gas provided in step (b) and/or the volume flow of the product gas removed in step (f),
**characterized in that**
- the reaction in step (e) occurs in at least a first catalyst bed and a second catalyst bed;
- a first substream of the gas mixture B is fed to the first catalyst bed and the gas mixture which leaves the first catalyst bed is fed to the second catalyst bed;
- a second substream of the gas mixture B is fed to the second catalyst bed with bypassing of the first catalyst bed;
- the relative volume flow ratio of the first substream to the second substream is increased during the first time interval; and
- the relative volume flow ratio of the first substream to the second substream is reduced again during the second time interval.

2. Process according to Claim 1, wherein the first substream of the gas mixture B makes up at least 40% by volume of the total amount of the gas mixture B.

3. Process according to either Claim 1 or 2 comprising complete stoppage of the volume flow of the first feed gas provided in step (a) and/or the volume flow of the second feed gas provided in step (b) and/or the volume flow of the product gas removed in step (f) during at least one third time interval.

4. Process according to Claim 3, wherein the gas mixture A and/or the gas mixture B and/or the gas mixture C enriched in product gas and/or the gas mixture D depleted in product gas
- are compressed; and/or
- are heated; and/or
- the gas mixture A and/or the gas mixture D depleted in product gas are at least partly conveyed so as to bypass the catalyst.

5. Process according to any of the preceding claims, wherein the first feed gas is hydrogen.

6. Process according to Claim 5, wherein the hydrogen is obtained by electrolysis of water.

7. Process according to any of the preceding claims, wherein the reaction in step (e) does not proceed to completion, so that the gas mixture C enriched in product gas comprises unreacted first feed gas and unreacted second feed gas.

8. Process according to any of the preceding claims, wherein the content of product gas in gas mixture D depleted in product gas is at least 0.5% by volume.

9. Process according to any of the preceding claims, wherein the second feed gas is nitrogen and the product gas is ammonia.

10. Process according to any of Claims 1 to 8, wherein the second feed gas is carbon monoxide or carbon dioxide.

11. Process according to any of the preceding claims, wherein the gas mixture A does not comprise any product gas.

12. Process according to any of Claims 1 to 11, wherein the removal of the product gas in step (f) is effected by condensation by means of a condenser and the exit temperature from the condenser is increased in the first time interval.

13. Plant for producing a product gas from a first feed gas and a second feed gas, in particular by the process according to any of preceding Claims 1 to 12, comprising the following components which are operatively connected to one another:
- a first inlet device configured for providing the first feed gas;
- a second inlet device configured for providing the second feed gas;
- a reactor (7) configured for synthesizing a product gas from the first feed gas and the second feed gas to produce a gas mixture C enriched in product gas under heterogeneous gas catalysis;
- a removal device (8) configured for removing product gas from the gas mixture C enriched in product gas with production of a gas mixture D depleted in product gas; and
- a control device which is configured for bringing the apparatus into a first operating state or into a second operating state, where the first operating state differs from the second operating state
(i) in terms of a smaller volume flow of the first feed gas which is provided by the first inlet device and/or a smaller volume flow of the second feed gas which is provided by the second inlet device and/or a smaller volume flow of the product gas which is removed by the removal device (8),
and/or
(ii) in terms of a smaller amount of product gas formed per unit time,
where the reactor (7) comprises at least one catalyst bed which has a radial distance from an outer shell of the reactor, which results in a free space between the catalyst bed and the outer shell of the reactor through which flow can occur, in such a way that a gas mixture entering the reactor (7) in the first operating state can flow essentially axially through the reactor without flowing through the catalyst bed, where the reactor (7) comprises at least two catalyst beds (7a, 7b, 7c) arranged in series in the axial direction, where means by means of which at least one substream of the inflowing gas mixture can flow directly, with bypassing of a first catalyst bed (7a), into a second catalyst bed (7b) located downstream of this first catalyst bed (7a).

14. Plant according to the preceding claim which comprises a conduit (10) which is configured for circulating a gas mixture from an outlet (11) of the reactor (7) to an inlet (12) of the reactor (7), where the circuit comprises a heating device (13) and the control device is configured so that in the first operating state at least part of the gas mixture C leaving the reactor (7) is conveyed from the outlet (11) of the reactor (7) through the conduit (10) to the inlet (12) of the reactor (7) and during this passage is heated in the heating device (13).

15. Use of a plant according to either Claim 13 or 14 in a process according to any of the preceding process claims.

## Revendications

1. Procédé de synthèse d'un produit gazeux, ledit procédé comprenant les étapes suivantes :
(a) fournir un premier éduit gazeux ;
(b) fournir un deuxième éduit gazeux ;
(c) combiner le premier éduit gazeux et le deuxième éduit gazeux pour obtenir un mélange gazeux A comprenant le premier éduit gazeux et le deuxième éduit gazeux ;
(d) combiner le mélange gazeux A avec un mélange gazeux circulant pour obtenir un mélange gazeux B comprenant le premier éduit gazeux, le deuxième éduit gazeux et le produit gazeux ;
(e) convertir le premier éduit gazeux et le deuxième éduit gazeux contenus dans le mélange gazeux B sur un catalyseur en produit gazeux pour produire un mélange gazeux C enrichi en produit gazeux ;
(f) retirer le produit gazeux du mélange gazeux C enrichi en produit gazeux pour produire un mélange gazeux D appauvri en produit gazeux ; et
(g) ramener au moins une partie du mélange gazeux D appauvri en produit gazeux en tant que mélange gazeux circulant à l'étape (d) ;
le débit volumique du premier éduit gazeux fourni à l'étape (a) et/ou le débit volumique du deuxième éduit gazeux fourni à l'étape (b) et/ou le débit volumique du produit gazeux retiré à l'étape (f) variant au cours du procédé, et comprenant en outre les étapes suivantes
- réduire temporairement la quantité de produit gazeux formée par unité de temps pendant un premier intervalle de temps par réduction du débit volumique du premier éduit gazeux fourni à l'étape (a) et/ou du débit volumique du deuxième éduit gazeux fourni à l'étape (b) et/ou du débit volumique du produit gazeux retiré l'étape (f) ; et
- augmenter la quantité de produit gazeux formé par unité de temps pendant un deuxième intervalle de temps par augmentation du débit volumique du premier éduit gazeux fourni à l'étape (a) et/ou du débit volumique du deuxième éduit gazeux fourni à l'étape (b) et/ou du débit volumique du produit gazeux retiré à l'étape (f),
**caractérisé en ce que**
- la réaction de l'étape (e) est effectuée dans au moins un premier lit de catalyseur et un deuxième lit de catalyseur ;
- un premier flux partiel du mélange gazeux B est amené au premier lit de catalyseur et le mélange gazeux qui quitte le premier lit de catalyseur est amené au deuxième lit de catalyseur ;
- un deuxième flux partiel du mélange gazeux B est amené au deuxième lit de catalyseur en contournant le premier lit de catalyseur ;
- le rapport relatif, en termes de débit volumique, du premier flux partiel au deuxième flux partiel est augmenté pendant le premier intervalle de temps ; et
- le rapport relatif, en termes de débit volumique, du premier flux partiel au deuxième flux partiel est à nouveau réduit pendant le deuxième intervalle de temps.

2. Procédé selon la revendication 1, le premier flux partiel du mélange gazeux B représentant au moins 40 % en volume de la quantité totale du mélange gazeux B.

3. Procédé selon l'une des revendications 1 et 2, comprenant le réglage complet du débit volumique du premier éduit gazeux fourni à l'étape (a) et/ou du débit volumique du deuxième éduit gazeux fourni à l'étape (b) et/ou du débit volumique du produit gazeux retiré de l'étape (f) pendant au moins un troisième intervalle de temps.

4. Procédé selon la revendication 3, le mélange gazeux A et/ou le mélange gazeux B et/ou le mélange gazeux C enrichi en produit gazeux et/ou le mélange gazeux D appauvri en produit gazeux
- étant comprimés ; et/ou
- étant chauffés ; et/ou
- le mélange gazeux A et/ou le mélange gazeux D appauvri en produit gazeux étant guidés en contournant au moins partiellement le catalyseur.

5. Procédé selon l'une des revendications précédentes, le premier éduit gazeux étant l'hydrogène.

6. Procédé selon la revendication 5, l'hydrogène étant obtenu par électrolyse de l'eau.

7. Procédé selon l'une des revendications précédentes, la réaction à l'étape (e) étant incomplète de sorte que le mélange gazeux C enrichi en produit gazeux comprenne du premier éduit gazeux n'ayant pas réagi et du deuxième éduit gazeux n'ayant pas réagi.

8. Procédé selon l'une des revendications précédentes, la teneur en produit gazeux dans un mélange gazeux D appauvri en produit gazeux étant d'au moins 0,5 % en volume.

9. Procédé selon l'une des revendications précédentes, le deuxième éduit gazeux étant l'azote et le produit gazeux étant l'ammoniac.

10. Procédé selon l'une des revendications 1 à 8, le deuxième éduit gazeux étant le monoxyde de carbone ou le dioxyde de carbone.

11. Procédé selon l'une des revendications précédentes, le mélange gazeux A ne comprenant aucun produit gazeux.

12. Procédé selon l'une des revendications 1 à 11, le produit gazeux étant retiré à l'étape (f) par condensation au moyen d'un condenseur et la température de sortie du condenseur étant augmentée dans le premier intervalle de temps.

13. Installation de production d'un produit gazeux à partir d'un premier éduit gazeux et d'un deuxième éduit gazeux, notamment selon le procédé de l'une des revendications 1 à 12 précédentes, ladite installation comprenant les composants suivants qui sont reliés fonctionnellement entre eux :
- un premier dispositif d'entrée conçu pour fournir le premier éduit gazeux ;
- un deuxième dispositif d'entrée conçu pour fournir le deuxième éduit gazeux ;
- un réacteur (7) conçu pour synthétiser un produit gazeux à partir du premier éduit gazeux et du deuxième éduit gazeux pour produire un mélange gazeux C enrichi en produit gazeux par catalyse gazeuse hétérogène ;
- un dispositif de retrait (8) conçu pour retirer le produit gazeux du mélange gazeux C enrichi en produit gazeux pour produire un mélange gazeux D appauvri en produit gazeux ; et
- un dispositif de commande conçu pour mettre le dispositif dans un premier état de fonctionnement ou dans un deuxième état de fonctionnement,
le premier état de fonctionnement différant du deuxième état de fonctionnement
(i) en ce qu'un débit volumique du premier éduit gazeux, fourni par le premier dispositif d'entrée, est plus faible et/ou en ce qu'un débit volumique du deuxième éduit gazeux, fourni par le deuxième dispositif d'entrée, est plus faible et/ou en ce qu'un débit volumique du produit gazeux, retiré par le dispositif de retrait (8), est plus faible
et/ou
(ii) en ce qu'une quantité de produit gazeux produite par unité de temps est plus faible,
le réacteur (7) comprenant au moins un lit de catalyseur qui est à une distance radiale d'une enveloppe extérieure du réacteur, ce qui se traduit par un espace d'écoulement libre entre le lit de catalyseur et l'enveloppe extérieure du réacteur, de manière à ce que dans le premier état de fonctionnement un mélange gazeux entrant dans le réacteur (7) puisse s'écouler sensiblement axialement à travers le réacteur sans s'écouler à travers le lit de catalyseur,
le réacteur (7) comprenant au moins deux lits de catalyseur (7a, 7b, 7c) disposés l'un derrière l'autre dans la direction axiale, des moyens étant prévus pour qu'au moins un flux partiel du mélange gazeux entrant puisse s'écouler directement dans un deuxième lit de catalyseur (7b) en aval d'un premier lit de catalyseur (7a) en contournant ce premier lit de catalyseur (7a).

14. Installation selon la revendication précédente, laquelle comprend une conduite (10) qui est conçue pour faire circuler un mélange gazeux depuis une sortie (11) du réacteur (7) jusqu'à une entrée (12) du réacteur (7), le circuit comprenant un élément chauffant (13), et le dispositif de commande étant conçu de telle sorte que dans le premier état de fonctionnement au moins une partie du mélange gazeux C quittant le réacteur (7) soit guidée de la sortie (11) du réacteur (7) jusqu'à l'entrée (12) du réacteur (7) par le biais de la conduite (10) et soit chauffée dans le dispositif de chauffage (13).

15. Utilisation d'un système selon l'une des revendications 13 et 14 dans un procédé selon l'une des revendications précédentes.
